(19) Europäisches Patentamt / European Patent Office / Office européen des brevets

(11) **EP 3 502 277 A1**

(12) **EUROPEAN PATENT APPLICATION**

(43) Date of publication:
**26.06.2019 Bulletin 2019/26**

(21) Application number: **17209972.3**

(22) Date of filing: **22.12.2017**

(51) Int Cl.:
***C12Q 1/6883*** *(2018.01)* ***C07K 14/435*** *(2006.01)*
***C07K 14/705*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Heinrich Heine Universität Düsseldorf**
**40225 Düsseldorf (DE)**

(72) Inventor: **Scholl, Ute**
**40225 Düsseldorf (DE)**

(74) Representative: **Kuttenkeuler, David**
**Boehmert & Boehmert**
**Anwaltspartnerschaft mbB**
**Pettenkoferstrasse 22**
**80336 München (DE)**

(54) **DIAGNOSIS OF PRIMARY ALDOSTERONISM**


(57) The present invention pertains to the identification of the gene *CLCN2* and its gene product, the membrane channel protein C1C-2, as main biomarker and causative factor for primary aldosteronism, and in particular familial forms of this disease. The invention provides specific mutations associated with the disorder, as well as nucleic acids comprising these variant sequences, primer and probes for their molecular detection and methods applying the compounds of the invention for a diagnosis of primary aldosteronism. In addition the invention provides novel treatments options for patients suffering from primary aldosteronism.


Figure 1:

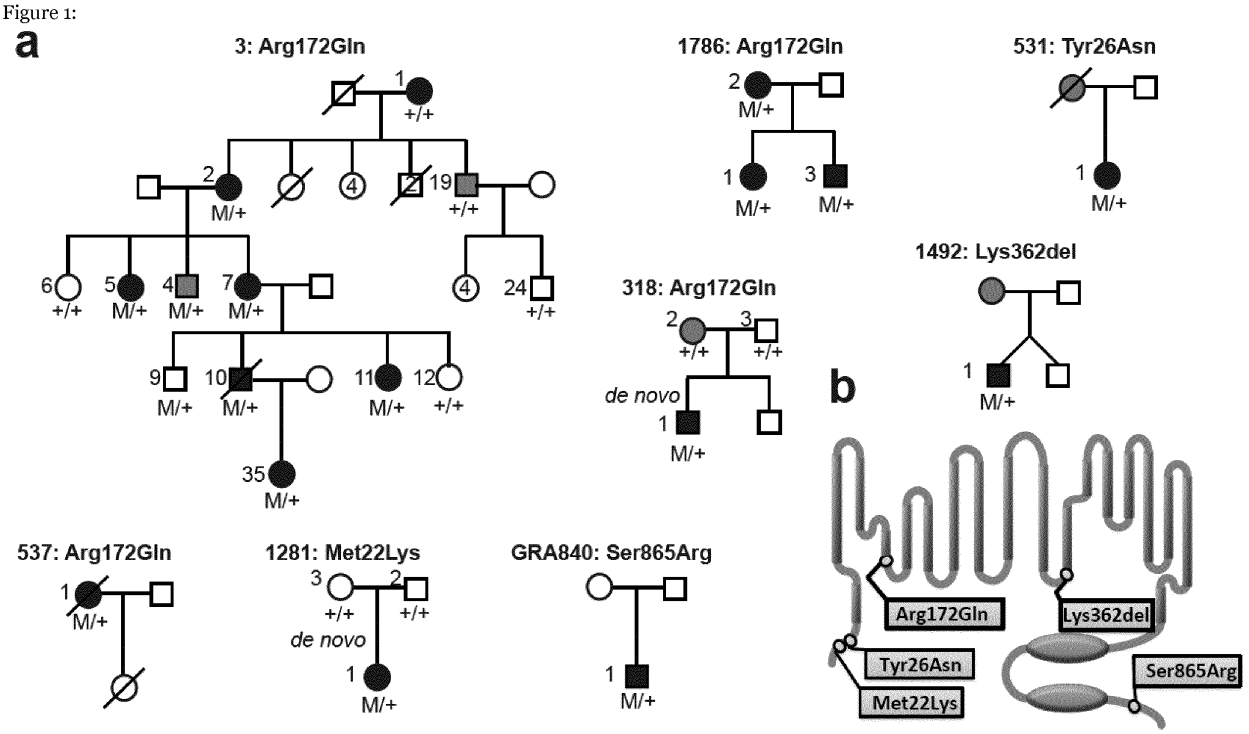




Printed by Jouve, 75001 PARIS (FR)

(Cont. next page)

Figure 1 cont.:

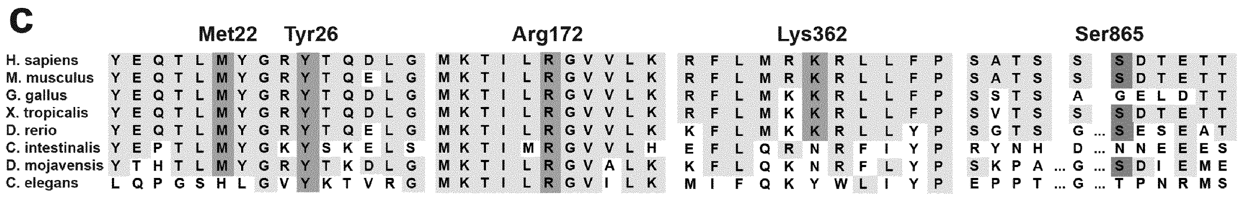

SEQUENCE LISTING

<110> Heinrich-Heine-Universitaet Duesseldorf

<120> DIAGNOSIS AND THERAPY OF PRIMARY ALDOSTERONISM

<130> U30828EP

<160> 34

<170> PatentIn version 3.5

<210> 1
<211> 898
<212> PRT
<213> homo sapiens

<400> 1

```
Met Ala Ala Ala Ala Ala Glu Glu Gly Met Glu Pro Arg Ala Leu Gln
1               5                   10                  15

Tyr Glu Gln Thr Leu Met Tyr Gly Arg Tyr Thr Gln Asp Leu Gly Ala
            20                  25                  30

Phe Ala Lys Glu Glu Ala Ala Arg Ile Arg Leu Gly Gly Pro Glu Pro
        35                  40                  45

Trp Lys Gly Pro Pro Ser Ser Arg Ala Ala Pro Glu Leu Leu Glu Tyr
    50                  55                  60

Gly Arg Ser Arg Cys Ala Arg Cys Arg Val Cys Ser Val Arg Cys His
65                  70                  75                  80

Lys Phe Leu Val Ser Arg Val Gly Glu Asp Trp Ile Phe Leu Val Leu
                85                  90                  95

Leu Gly Leu Leu Met Ala Leu Val Ser Trp Val Met Asp Tyr Ala Ile
            100                 105                 110

Ala Ala Cys Leu Gln Ala Gln Gln Trp Met Ser Arg Gly Leu Asn Thr
        115                 120                 125

Ser Ile Leu Leu Gln Tyr Leu Ala Trp Val Thr Tyr Pro Val Val Leu
    130                 135                 140

Ile Thr Phe Ser Ala Gly Phe Thr Gln Ile Leu Ala Pro Gln Ala Val
145                 150                 155                 160

Gly Ser Gly Ile Pro Glu Met Lys Thr Ile Leu Arg Gly Val Val Leu
                165                 170                 175
```

```
Lys Glu Tyr Leu Thr Leu Lys Thr Phe Ile Ala Lys Val Ile Gly Leu
            180                 185                 190

Thr Cys Ala Leu Gly Ser Gly Met Pro Leu Gly Lys Glu Gly Pro Phe
        195                 200                 205

Val His Ile Ala Ser Met Cys Ala Ala Leu Leu Ser Lys Phe Leu Ser
    210                 215                 220

Leu Phe Gly Gly Ile Tyr Glu Asn Glu Ser Arg Asn Thr Glu Met Leu
225                 230                 235                 240

Ala Ala Ala Cys Ala Val Gly Val Gly Cys Cys Phe Ala Ala Pro Ile
                245                 250                 255

Gly Gly Val Leu Phe Ser Ile Glu Val Thr Ser Thr Phe Phe Ala Val
            260                 265                 270

Arg Asn Tyr Trp Arg Gly Phe Phe Ala Ala Thr Phe Ser Ala Phe Ile
        275                 280                 285

Phe Arg Val Leu Ala Val Trp Asn Arg Asp Glu Glu Thr Ile Thr Ala
    290                 295                 300

Leu Phe Lys Thr Arg Phe Arg Leu Asp Phe Pro Phe Asp Leu Gln Glu
305                 310                 315                 320

Leu Pro Ala Phe Ala Val Ile Gly Ile Ala Ser Gly Phe Gly Gly Ala
                325                 330                 335

Leu Phe Val Tyr Leu Asn Arg Lys Ile Val Gln Val Met Arg Lys Gln
            340                 345                 350

Lys Thr Ile Asn Arg Phe Leu Met Arg Lys Arg Leu Leu Phe Pro Ala
        355                 360                 365

Leu Val Thr Leu Leu Ile Ser Thr Leu Thr Phe Pro Pro Gly Phe Gly
    370                 375                 380

Gln Phe Met Ala Gly Gln Leu Ser Gln Lys Glu Thr Leu Val Thr Leu
385                 390                 395                 400

Phe Asp Asn Arg Thr Trp Val Arg Gln Gly Leu Val Glu Glu Leu Glu
                405                 410                 415

Pro Pro Ser Thr Ser Gln Ala Trp Asn Pro Pro Arg Ala Asn Val Phe
            420                 425                 430
```

```
Leu Thr Leu Val Ile Phe Ile Leu Met Lys Phe Trp Met Ser Ala Leu
        435                 440                 445

Ala Thr Thr Ile Pro Val Pro Cys Gly Ala Phe Met Pro Val Phe Val
    450                 455                 460

Ile Gly Ala Ala Phe Gly Arg Leu Val Gly Glu Ser Met Ala Ala Trp
465                 470                 475                 480

Phe Pro Asp Gly Ile His Thr Asp Ser Ser Thr Tyr Arg Ile Val Pro
                485                 490                 495

Gly Gly Tyr Ala Val Val Gly Ala Ala Ala Leu Ala Gly Ala Val Thr
            500                 505                 510

His Thr Val Ser Thr Ala Val Ile Val Phe Glu Leu Thr Gly Gln Ile
        515                 520                 525

Ala His Ile Leu Pro Val Met Ile Ala Val Ile Leu Ala Asn Ala Val
    530                 535                 540

Ala Gln Ser Leu Gln Pro Ser Leu Tyr Asp Ser Ile Ile Arg Ile Lys
545                 550                 555                 560

Lys Leu Pro Tyr Leu Pro Glu Leu Gly Trp Gly Arg His Gln Gln Tyr
                565                 570                 575

Arg Val Arg Val Glu Asp Ile Met Val Arg Asp Val Pro His Val Ala
            580                 585                 590

Leu Ser Cys Thr Phe Arg Asp Leu Arg Leu Ala Leu His Arg Thr Lys
        595                 600                 605

Gly Arg Met Leu Ala Leu Val Glu Ser Pro Glu Ser Met Ile Leu Leu
    610                 615                 620

Gly Ser Ile Glu Arg Ser Gln Val Val Ala Leu Leu Gly Ala Gln Leu
625                 630                 635                 640

Ser Pro Ala Arg Arg Arg Gln His Met Gln Glu Arg Arg Ala Thr Gln
                645                 650                 655

Thr Ser Pro Leu Ser Asp Gln Glu Gly Pro Pro Thr Pro Glu Ala Ser
            660                 665                 670

Val Cys Phe Gln Val Asn Thr Glu Asp Ser Ala Phe Pro Ala Ala Arg
        675                 680                 685
```

```
Gly Glu Thr His Lys Pro Leu Lys Pro Ala Leu Lys Arg Gly Pro Ser
    690                 695                 700

Val Thr Arg Asn Leu Gly Glu Ser Pro Thr Gly Ser Ala Glu Ser Ala
705                 710                 715                 720

Gly Ile Ala Leu Arg Ser Leu Phe Cys Gly Ser Pro Pro Pro Glu Ala
                725                 730                 735

Ala Ser Glu Lys Leu Glu Ser Cys Glu Lys Arg Lys Leu Lys Arg Val
            740                 745                 750

Arg Ile Ser Leu Ala Ser Asp Ala Asp Leu Glu Gly Glu Met Ser Pro
        755                 760                 765

Glu Glu Ile Leu Glu Trp Glu Glu Gln Gln Leu Asp Glu Pro Val Asn
    770                 775                 780

Phe Ser Asp Cys Lys Ile Asp Pro Ala Pro Phe Gln Leu Val Glu Arg
785                 790                 795                 800

Thr Ser Leu His Lys Thr His Thr Ile Phe Ser Leu Leu Gly Val Asp
                805                 810                 815

His Ala Tyr Val Thr Ser Ile Gly Arg Leu Ile Gly Ile Val Thr Leu
            820                 825                 830

Lys Glu Leu Arg Lys Ala Ile Glu Gly Ser Val Thr Ala Gln Gly Val
        835                 840                 845

Lys Val Arg Pro Pro Leu Ala Ser Phe Arg Asp Ser Ala Thr Ser Ser
    850                 855                 860

Ser Asp Thr Glu Thr Thr Glu Val His Ala Leu Trp Gly Pro His Ser
865                 870                 875                 880

Arg His Gly Leu Pro Arg Glu Gly Ser Pro Ser Asp Ser Asp Asp Lys
                885                 890                 895

Cys Gln


<210>  2
<211>  2697
<212>  DNA
<213>  homo sapiens
```

<400> 2
atggcggccg cggcggcgga ggaagggatg gagccacggg cgctgcagta cgagcagacc 60

ctgatgtatg gccggtacac tcaggacctt ggggcctttg ccaaagagga agctgctcgg 120

attcgcctgg gagggcctga accctggaaa ggtccccctt cctctcgggc tgccccagag 180

ctcttggaat atggacggag ccgttgcgcc cgatgccgcg tctgttctgt ccgctgccac 240

aagttcctag tatccagggt tggtgaagat tggatcttcc tggtcctgct ggggcttctc 300

atggcattgg tcagctgggt catggactat gccattgctg cctgtctgca agcccagcag 360

tggatgtccc ggggcttgaa caccagcatc ttgctccagt acctggcctg ggtcacctac 420

cctgttgtcc tcatcacttt ctcagccgga ttcacacaga tcctggcccc tcaggctgtc 480

ggctctggca tccctgagat gaagaccatc ttgcggggag tggtgctgaa agaatacctc 540

acactcaaga cctttatagc taaggtcatt gggctgacct gcgccctagg cagcgggatg 600

ccgcttggca aagagggccc ttttgtgcat atcgcaagca tgtgtgctgc ccttctcagc 660

aagttcctct ccctctttgg gggtatctat gagaatgaat cccggaacac agagatgctg 720

gctgccgcct gtgccgtggg ggtgggctgc tgcttcgcgg cacctattgg aggcgtcctc 780

ttcagcatcg aggtcacctc caccttcttt gcagtgcgga actactggcg gggcttcttc 840

gctgccacct tcagtgcctt catcttccgg gtcttggcag tctggaaccg ggatgaagag 900

actattacag ccctcttcaa aacccgattc cggctcgact tccccttga cctgcaggag 960

ctgccagcct ttgctgtcat tggtattgct agtggcttcg gtggagccct ctttgtctac 1020

ctgaaccgga agattgtcca ggtgatgcgg aagcagaaaa ccatcaatcg cttcctcatg 1080

aggaaacgcc tgctcttccc ggctctggtg accctgctca tctccacgct gaccttcccc 1140

cctggctttg gacagttcat ggctggacag ctctcacaga aagagacgct ggtcaccctg 1200

tttgacaatc ggacgtgggt ccgccagggc ctggtggagg agctagaacc acccagcacc 1260

tcacaggcct ggaacccacc acgtgccaac gtcttcctca ccctggtcat cttcattctc 1320

atgaagttct ggatgtctgc actggccacc accatcccag ttccctgtgg ggccttcatg 1380

cctgtctttg tcattggagc agcatttggg cgtctggtgg gtgaaagcat ggctgcctgg 1440

ttcccagatg gaattcatac ggacagcagc acctaccgga ttgtgcctgg gggctacgct 1500

gtggtcgggg cagctgcgct ggcaggagcg gtgacacaca cagtgtccac ggctgtgatc 1560

gtgttcgagc tcacaggcca gattgcccac atcctgcctg tcatgatcgc cgtcatcctg 1620

gccaacgctg tcgcccagag tctgcagccc tccctctatg acagcatcat ccgaatcaag 1680

aaactgccct acctgcctga gctcggctgg ggccgccacc agcagtaccg ggtgcgtgtg 1740

gaggacatca tggtgcggga tgttccccat gtggccctca gctgcacctt ccgggacctg 1800

cgtttggcac tgcacaggac caagggccga atgctggccc tagtggagtc ccctgagtcc 1860

atgattctgc tgggctccat cgagcgttca caggtggtgg cattgttggg ggcccagctg 1920

agcccagccc gccggcggca gcacatgcag gagcgcagag ccacccagac ctctccacta 1980

tctgatcagg agggtccccc taccccctgag gcttctgtct gcttccaggt gaacacagaa 2040

gactcagcct tcccagcagc ccggggggag acccacaagc ccctaaagcc tgcactcaag 2100

agggggccca gtgtcaccag gaacctcgga gagagtccca cagggagcgc agagtcggca 2160

ggcatcgccc tccggagcct cttctgtggc agtccacccc ctgaggctgc ttcggagaag 2220

ttggaatcct gtgagaagcg caagctgaag cgtgtccgaa tctccctggc aagtgacgcg 2280

gacctggaag gcgagatgag ccctgaagag attctggagt gggaggagca gcaactagat 2340

gaacctgtca acttcagtga ctgcaaaatt gatcctgctc ccttccagct ggtggagcgg 2400

acctctttgc acaagactca cactatcttc tcactgctgg gagtggacca tgcttatgtc 2460

accagtattg gcagactcat tggaatcgtt actctaaagg agctccggaa ggccatcgag 2520

ggctctgtca cagcacaggg tgtgaaagtc cggccgcccc tcgccagctt ccgagacagt 2580

gccaccagca gcagtgacac ggagaccact gaggtgcatg cactctgggg gccccactcc 2640

cgtcatggcc tcccccggga gggcagccct tccgacagcg acgacaaatg ccaatga 2697

<210> 3
<211> 15
<212> PRT
<213> Homo sapiens

<400> 3

Tyr Glu Gln Thr Leu Met Tyr Gly Arg Tyr Thr Gln Asp Leu Gly
1 5 10 15

<210> 4
<211> 15
<212> PRT
<213> Mus musculus

<400> 4

Tyr Glu Gln Thr Leu Met Tyr Gly Arg Tyr Thr Gln Glu Leu Gly
1 5 10 15

<210> 5
<211> 15
<212> PRT
<213> Gallus gallus

<400> 5

Tyr Glu Gln Thr Leu Met Tyr Gly Arg Tyr Thr Gln Asp Leu Gly
1 5 10 15

<210> 6

<211>  15
<212>  PRT
<213>  X. tropicalis

<400>  6

Tyr Glu Gln Thr Leu Met Tyr Gly Arg Tyr Thr Gln Asp Leu Gly
1               5                   10                  15

<210>  7
<211>  15
<212>  PRT
<213>  D. rerio

<400>  7

Tyr Glu Gln Thr Leu Met Tyr Gly Arg Tyr Thr Gln Glu Leu Gly
1               5                   10                  15

<210>  8
<211>  15
<212>  PRT
<213>  C. intestinalis

<400>  8

Tyr Glu Pro Thr Leu Met Tyr Gly Lys Tyr Ser Lys Glu Leu Ser
1               5                   10                  15

<210>  9
<211>  15
<212>  PRT
<213>  D. mojavensis

<400>  9

Tyr Thr His Thr Leu Met Tyr Gly Arg Tyr Thr Lys Asp Leu Gly
1               5                   10                  15

<210>  10
<211>  15
<212>  PRT
<213>  C. elegans

<400>  10

Leu Gln Pro Gly Ser His Leu Gly Val Tyr Lys Thr Val Arg Gly
1               5                   10                  15

<210>  11
<211>  11
<212>  PRT
<213>  Homo sapiens

<400>  11

Met Lys Thr Ile Leu Arg Gly Val Val Leu Lys
1               5                   10

<210> 12
<211> 11
<212> PRT
<213> Mus musculus

<400> 12

Met Lys Thr Ile Leu Arg Gly Val Val Leu Lys
1 5 10

<210> 13
<211> 11
<212> PRT
<213> Gallus gallus

<400> 13

Met Lys Thr Ile Leu Arg Gly Val Val Leu Lys
1 5 10

<210> 14
<211> 11
<212> PRT
<213> X. tropicalis

<400> 14

Met Lys Thr Ile Leu Arg Gly Val Val Leu Lys
1 5 10

<210> 15
<211> 11
<212> PRT
<213> D. rerio

<400> 15

Met Lys Thr Ile Leu Arg Gly Val Val Leu Lys
1 5 10

<210> 16
<211> 11
<212> PRT
<213> C. intestinalis

<400> 16

Met Lys Thr Ile Met Arg Gly Val Val Leu His
1 5 10

<210> 17
<211> 11
<212> PRT
<213> D. mojavensis

<400> 17

Met Lys Thr Ile Leu Arg Gly Val Ala Leu Lys
1 5 10

<210> 18
<211> 11
<212> PRT
<213> C. elegans

<400> 18

Met Lys Thr Ile Leu Arg Gly Val Ile Leu Lys
1 5 10

<210> 19
<211> 11
<212> PRT
<213> Homo sapiens

<400> 19

Arg Phe Leu Met Arg Lys Arg Leu Leu Phe Pro
1 5 10

<210> 20
<211> 11
<212> PRT
<213> Mus musculus

<400> 20

Arg Phe Leu Met Arg Lys Arg Leu Leu Phe Pro
1 5 10

<210> 21
<211> 11
<212> PRT
<213> Gallus gallus

<400> 21

Arg Phe Leu Met Lys Lys Arg Leu Leu Phe Pro
1 5 10

<210> 22
<211> 11
<212> PRT
<213> X. tropicalis

<400> 22

Arg Phe Leu Met Lys Lys Arg Leu Leu Phe Pro
1 5 10

<210> 23
<211> 11
<212> PRT

<213> D. rerio

<400> 23

Lys Phe Leu Met Lys Lys Arg Leu Leu Tyr Pro
1 5 10

<210> 24
<211> 11
<212> PRT
<213> C. intestinalis

<400> 24

Glu Phe Leu Gln Arg Asn Arg Phe Ile Tyr Pro
1 5 10

<210> 25
<211> 11
<212> PRT
<213> D. mojavensis

<400> 25

Lys Phe Leu Gln Lys Asn Arg Phe Leu Tyr Pro
1 5 10

<210> 26
<211> 11
<212> PRT
<213> C. elegans

<400> 26

Met Ile Phe Gln Lys Tyr Trp Leu Ile Tyr Pro
1 5 10

<210> 27
<211> 11
<212> PRT
<213> Homo sapiens

<400> 27

Ser Ala Thr Ser Ser Ser Asp Thr Glu Thr Thr
1 5 10

<210> 28
<211> 11
<212> PRT
<213> Mus musculus

<400> 28

Ser Ala Thr Ser Ser Ser Asp Thr Glu Thr Thr
1 5 10

<210> 29
<211> 11
<212> PRT
<213> Gallus gallus

<400> 29

Ser Ser Thr Ser Ala Gly Glu Leu Asp Thr Thr
1 5 10

<210> 30
<211> 11
<212> PRT
<213> X. tropicalis

<400> 30

Ser Val Thr Ser Ser Ser Asp Thr Glu Thr Thr
1 5 10

<210> 31
<211> 11
<212> PRT
<213> D. RERIO

<400> 31

Ser Gly Thr Ser Gly Ser Glu Ser Glu Ala Thr
1 5 10

<210> 32
<211> 11
<212> PRT
<213> C. intestinalis

<400> 32

Arg Tyr Asn His Asp Asn Asn Glu Glu Glu Ser
1 5 10

<210> 33
<211> 11
<212> PRT
<213> D. mojavensis

<400> 33

Ser Lys Pro Ala Gly Ser Asp Ile Glu Met Glu
1 5 10

<210> 34
<211> 11
<212> PRT
<213> C. elegans

<400> 34

Glu Pro Pro Thr Gly Thr Pro Asn Arg Met Ser

1 5 10

**Description**

FIELD OF THE INVENTION

The present invention pertains to the identification of the gene *CLCN2* and its gene product, the membrane channel protein ClC-2, as main biomarker and causative factor for primary aldosteronism, and in particular familial forms of this disease. The invention provides specific mutations associated with the disorder, as well as nucleic acids comprising these variant sequences, primer and probes for their molecular detection and methods applying the compounds of the invention for a diagnosis of primary aldosteronism. In addition the invention provides novel treatments options for patients suffering from primary aldosteronism.

DESCRIPTION

More than 1.1 billion people worldwide have hypertension, the single largest cause of premature mortality. About 6% of hypertensive patients in primary care have primary aldosteronism (Monticone, S. et al. J Am Coll Cardiol 69, 1811-1820 (2017)) with higher frequencies among patients with severe hypertension. The plasma aldosterone level in primary aldosteronism is constitutively elevated despite low levels of the normal upstream regulator renin; hypokalemia is variable. Aldosterone-producing adrenal adenomas (APAs) and idiopathic hyperaldosteronism are common causes of primary aldosteronism. Somatic mutations in *KCNJ5, CACNA1D, ATP1A1,* or *ATP2B3* that cause increased glomerulosa cell $Ca^{2+}$ are sufficient for producing APAs; germline mutations alter *CYP11B211* in glucocorticoid-remediable aldosteronism (GRA, FH-I), *KCNJ5* in FH-III, *CACNA1H* in FH-IV, and *CACNA1D* in PASNA syndrome. These mutations define a common pathway for induction of aldosterone biosynthesis - glomerulosa cell membrane depolarization activates voltage-gated $Ca^{2+}$ channels, which induces the rate-limiting enzyme for aldosterone biosynthesis, aldosterone synthase (CYP11B2), along with other enzymes in the biosynthetic pathway; increased mitochondrial $Ca^{2+}$ may also contribute.

In 1992, Stowasser et al. described a multiplex kindred featuring autosomal dominant primary aldosteronism that was clinically distinct from GRA, the only dominant syndrome then known, and hence called it FH-II (Stowasser, M. et al. Familial hyperaldosteronism type II: five families with a new variety of primary aldosteronism. Clin Exp Pharmacol Physiol 19, 319-22 (1992)). The responsible gene in this kindred has not been identified. Hence, the present invention seeks to unravel the genetic causes of primary aldosteronism and to provide novel means for diagnosing and treating primary aldosteronism.

The above problem is solved in a first aspect by an isolated nucleic acid molecule (i) comprising a sequence encoding for chloride volt-age-gated channel 2 (*CLCN2*) protein having the amino acid sequence shown in SEQ ID NO: 1, with at least one amino acid sequence variation compared to that sequence; or (ii) comprising a sequence according to SEQ ID NO: 2 with at least one nucleic acid sequence variation compared to that sequence.

In another aspect the problem of the invention is solved by an isolated nucleic acid molecule encoding for CLCN2, preferably a nucleic acid molecule comprising the *CLCN2* gene or *CLCN2* mRNA, wherein the isolated nucleic acid molecule is characterized by at least one mutation compared to the wild-type sequences of human *CLCN2.* Preferably the at least one mutation is a mutation which alters CLCN2 structure, stability, function or expression. As such the at least one mutation according to the invention may be located in the isolated nucleic acid in the open reading frame (ORF), or an intronic sequence, or any 3 prime or 5 prime regulatory sequence. Preferred is however that the mutation is located in the ORF and results, when the sequence is expressed, into a CLCN2 protein having a mutated amino acid sequence.

In the context of the present invention it was surprisingly discovered that the candidate gene *CLCN2* is significantly associated with primary aldosteronism. The gene encodes the voltage- gated chloride channel ClC-2 (Thiemann (1992), Nature 365,57-60 ; Cid (1995), Hum. Mol. Genet. 4,407-413). ClC-2 is strongly expressed in brain, in particular in - aminobutyric acid (GABA) -inhibited neurons (Smith (1995), J. Neurosci. 15,4057- 4067; Sik (2000), Neuroscience 101,51-65). In context of the invention when referring to the gene or gene sequence the disclosure will use the *"CLCN2" or "CLCN2* gene", or similar expressions. When referring to the protein encoded by *CLCN2*, the present disclosure will refer to "ClC-2", "CLCN2" or "CLCN2 protein" or similar expressions.

Preferred in context of the invention is that the variant or mutation of the invention is a *CLCN2* gain-of-function mutation. In particular preferred is that the mutation or variation leads to a ClC-2 channel with an electrophysiologic impact and/or, producing membrane depolarization and increasing CYP11B2 expression.

The terms "mutation" and "variant" shall be used interchangingly in context of the present invention.

Preferably, the at least one amino acid sequence variation is at position Arg172, Met22, Tyr26, Ser865, and/or Lys362 in SEQ ID NO: 1, the amino acid sequence of CLCN2.

Moreover, an isolated nucleic acid sequence of the invention is preferred, wherein the at least one amino acid sequence variation is selected from Arg172Gln, Met22Lys, Tyr26Asn, Ser865Arg, and/or Lys362del. The term "del" in this context refers to a deletion of the respective amino acid.

The invention in other embodiments also provides an isolated nucleic acid primer or probe having a nucleic acid sequence identical to, or complementary to, or binding to under hybridizing conditions, a stretch of 8 to 20 consecutive nucleotides of the isolated nucleic acid molecule according to the herein disclosed and claimed isolated nucleic acid, and wherein the stretch of 8 to 20 consecutive nucleotides comprises the codon sequence of the at least one amino acid sequence variation or nucleic acid sequence variation.

The term "hybridizing conditions" is intended to mean those conditions of time, temperature, and pH, and the necessary amounts and concentrations of reactants and reagents, sufficient to allow at least a portion of complementary sequences to anneal with each other. As is well known in the art, the time, temperature, and pH conditions required to accomplish hybridization depend on the size of the oligonucleotide probe or primer to be hybridized, the degree of complementarity between the oligonucleotide probe or primer and the target, and the presence of other materials in the hybridization reaction admixture. The actual conditions necessary for each hybridization step are well known in the art or can be determined without undue experimentation.

In one additional aspect the problem of the invention is solved by a method for detecting the presence of primary aldosteronism (PA) in a subject, the method comprising the steps of:

a. Providing a biological sample of the subject, and
b. detecting in the biological sample the presence or absence of one or more polymorphic sites in the *CLCN2* gene or CLCN2 protein, and

wherein the presence of the polymorphic site is indicative of the subject suffering from PA.

In one additional aspect the invention provides a method for detecting the genetic sequence of *CLCN2*, or of the amino acid sequence of ClC-2, in a subject, the method comprising the steps of

a. Providing a biological sample of the subject, and
b. detecting in the biological sample the genetic sequence of *CLCN2*, or of the amino acid sequence of ClC-2, by detecting the presence or absence of one or more polymorphic sites within the *CLCN2* nucleic acid sequence or ClC-2 amino acid sequence respectively.

Preferably the one or more polymorphic site(s) in the *CLCN2* gene may encode(s) for, or wherein the one or more polymorphic sites in the CLCN2 protein is, at least one amino acid residue variation in the CLCN2 protein (SEQ ID NO: 1). The at least one amino acid residue variation in the CLCN2 protein is preferably at position Arg172, Met22, Tyr26, Ser865, and/or Lys362 in SEQ ID NO: 1. Even more preferably the at least one amino acid residue variation in the CLCN2 protein is selected from Arg172Gln, Met22Lys, Tyr26Asn, Ser865Arg, and/or Lys362del.

The present invention pertains to the treatment and diagnosis of PA. Preferred is however, that the PA is early onset PA and/or is familial hyperaldosteronism type II (FH-II). This applies for all embodiments and aspects of the herein disclosed invention.

In the method for detecting the presence of primary aldosteronism (PA) in a subject according to the invention, step (b) preferably comprises the use of an isolated nucleic acid primer or probe as described herein before. Such a use may include a use in polymerase chain reactions (PCR) and/or *in situ* or other hybridization based detection methods.

In context of the present invention a biological sample is a sample containing genetic material of the subject - such a genomic DNA or mRNA, and/or wherein the sample contains proteinaceous material. A preferred biological sample is a liquid or tissue sample, such as whole blood, blood plasma, urine, tears, semen, saliva, buccal mucosa, interstitial fluid, lymph fluid, meningeal fluid, amniotic fluid, glandular fluid, sputum, feces, perspiration, mucous, vaginal secretion, cerebrospinal fluid, hair, skin, fecal material, wound exudate, wound homogenate, and wound fluid.

In some embodiments of the invention the method for detecting PA in a subject involves within step (b) the detection of the binding of an antibody to the polymorphic site(s) in the CLCN2 protein (ClC-2), wherein said antibody is specific for to the polymorphic site(s) in the CLCN2 protein (ClC-2). Preferably the binding occurs at position Arg172, Met22, Tyr26, Ser865, and/or Lys362, of the variant CLCN2 protein.

In another aspect the invention also pertains to an isolated protein comprising an amino acid sequence encoded by the isolated nucleic acid as described herein above.

In another aspect the invention also provides an antigen binding protein specifically binding to an isolated protein comprising an amino acid sequence encoded by the isolated nucleic acid as described herein above. Preferred antigen binding proteins of the invention specifically bind ClC-2 at position Arg172, Met22, Tyr26, Ser865, and/or Lys362, of the protein. The antibody may either specifically bind the mutated or the wild-type protein. Both possibilities will allow the skilled artisan to use the antigen binding protein to distinguish between the two version of ClC-2, and *CLCN2* respectively.

The antigen binding protein of the invention is in preferred embodiments an antibody, or an antigen binding fragment thereof. More preferably the antibody is a monoclonal antibody.

Also provided by the invention is a diagnostic kit, comprising an isolated nucleic acid according, a primer or probe, an isolated protein, or an antigen binding protein as described herein before. The diagnostic kit of the invention is preferably for use in a detection method as described herein before. Hence, also provided is the use of the diagnostic kit in such a method. The kit of the invention in some embodiments may additionally comprise instructions for its use, and/or any buffers, reagents and/or tools suitable for performing the methods of the invention.

In one additional aspect the invention further provides a method for treating a subject suffering for or expected to suffer from PA, the method comprising

a. Providing a biological sample of the subject, and
b. detecting in the biological sample the presence or absence of one or more polymorphic sites in the *CLCN2* gene or CLCN2 protein, and

wherein the presence of the polymorphic site results in the administration of a therapeutic of PA to the subject, and wherein the absence of the polymorphic site results in omission of the administration of a therapeutic of PA to the subject.

In another aspect the invention provides a method of treating a subject suffering from PA, the method comprising the administration of a compound to the subject in an amount therapeutically effective to treat the PA in the subject, and wherein the compound is an agent reducing the function, expression or activity of mutated ClC-2 in the subject. In this aspect, the subject is characterized by having a genetic mutation or variation in the *CLCN2* gen, preferably resulting in a mutated ClC-2 protein. More preferably the mutation or variation is a gain-of-function mutation, as described herein before. Exemplary compounds may include gene-editing compounds such as a guide RNA or guide DNA comprising a sequence for targeting the mutation in the *CLCN2* gene and editing it to restore *CLCN2* wild-type sequence. Gene-editing approaches are well known in the art and include, but are not limited to, the CIRSPR/CAS9 or similar systems.

The term "gene editing " and its grammatical equivalents as used herein can refer to genetic engineering in which one or more nucleotides are inserted, replaced, or removed from a genome. Gene editing can be performed using a nuclease (e.g. , a natural -existing nuclease or an artificially engineered nuclease).

A "CRISPR," "CRISPR system," and their grammatical equivalents can include a non - coding RNA molecule (e.g. , guide RNA) that binds to DNA and Cas proteins (e.g. , Cas9) with nuclease functionality (e.g. , two nuclease domains). See, e.g., Sander, J.D., et al , "CRISPR-Cas systems for editing, regulating and targeting genomes," Nature Biotechnology, 32:347-355 (2014); see also e.g. , Hsu, P.D., et al , "Development and applications of CRISPR-Cas9 for genome engineering," Cell 157(6): 1262-1278 (2014).

However, the invention shall also include any other compounds such as a polypeptide, a peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules. The compounds for use in a method of the invention shall have the ability to reduce the function, expression or activity of ClC-2, and preferably restore the gain-of-function mutation induced phenotype to the wild-type situation, such as a reduced membrane depolarization and decreasing CYP11B2 expression in a target cell of the subject.

The present invention will now be further described in the following examples with reference to the accompanying figures and sequences, nevertheless, without being limited thereto. For the purposes of the present invention, all references as cited herein are incorporated by reference in their entireties. In the Figures:

**Figure 1:** Kindreds with Hypertension and Primary Aldosteronism with *CLCN2* Mutations. (a) Pedigrees of eight kindreds with primary aldosteronism and hypertension, with indicated novel *CLCN2* variants. Filled black symbols denote subjects with primary aldosteronism, and filled grey symbols denote subjects with early-onset hypertension or borderline elevated ARRs. Genotypes are shown beneath each symbol, M/+ denotes the indicated novel CLCN2 variant in the heterozygous state, and +/+ denotes homozygous wildtype sequence. (b) Position of the indicated variants in the N-terminus, D helix, K helix and C-terminus of the ClC-2 chloride channel encoded by *CLCN2.* Red ellipses represent C-terminal CBS domains. (c) Conservation of the respective amino acid positions (SEQ ID NO: 3 to 34) among orthologous species (*H. sapiens,* human; *M. musculus,* mouse; *G. gallus,* chicken; *X. tropicalis,* western clawed frog; *D. rerio,* zebrafish; *C. intestinalis,* vase tunicate; *D. mojavensis,* fruit fly; *C. elegans,* roundworm).

**Figure 2:** Expression of ClC-2 in Human Adrenal Gland. (a) Section of human adrenal cortex (C, capsule; G, glomerulosa; F, fasciculata; R, reticularis) stained by immunohistochemistry and counterstained with hematoxylin.

One of two technical replicates is shown. Left, anti-ClC-2; middle, control preincubation of the anti-ClC-2 antibody with the antigenic peptide; right, anti-Dab2 as marker of the adrenal zona glomerulosa. The comparison of the three panels demonstrates specific expression of ClC-2, predominantly in the zona glomerulosa. Scale bars represent 200 μm. (b) Higher magnifications of the zona glomerulosa, scale bars represent 100 μm.

**Figure 3:** ***CLCN2* Mutations Increase Excitatory Anion Efflux by Modifying the Voltage Dependence of Channel Opening. (a)** Representative mouse adrenal gland section loaded with MQAE at 37°C. Short lifetimes (red, 1 ns) indicate high, long lifetimes (blue, 4 ns) low intracellular chloride concentrations. Scale bar, 10 μm. **(b)** Insert, calibration curve of MQAE fluorescence lifetimes in cells with preset intracellular chloride concentrations. A kernel density estimation of intracellular chloride concentrations for glomerulosa cells was obtained (Gaussian kernel, bandwidth=8.0 as determined by Scott's rule). Median intracellular chloride concentration was 74.7 mM (300 cells, 12 slices, five animals). Box, interquartile range; whiskers, 1.5x interquartile range; line, median. **(c)** Whole-cell patch clamp recordings of representative ClC-$2^{WT}$ and ClC-$2^{MUT}$ and voltage protocol (150 mM $[Cl^-]_{out}$, 75 mM $[Cl^-]_{int}$, see Online Methods for solutions) are shown. **(d)** Time constants for representative ClC-$2^{WT}$ and ClC-$2^{MUT}$ with mean values ± 95% confidence intervals are shown. **(e)** and **(f)** Mean relative open probabilities (e) and common gate open probabilities (f) were fit with a Boltzmann function (bold lines) with 95% confidence intervals as calculated from a bootstrap resampling (translucent areas). Open probabilities of mutant channels are significantly higher at the glomerulosa resting potential of ~-80 mV (WT, 0.22±0.02 (n=11); p.Arg172Gln, 0.45±0.02 (n=13; p<0.001 vs. WT); p.Ser865Arg, 0.33±0.02 (n=12; p<0.001 vs. WT); all mean±SEM, one-way ANOVA; F=36.307; d.f.=5). The insert in (e) demonstrates the shift in voltage activation as assessed by the point of half maximal activation. **, p<0.01.

**Figure 4:** ClC-2 Increases Aldosterone Synthase Expression in H295R cells. (a) RNA sequencing of H295R cells transfected with *CLCN2* (WT or p.Arg172Gln), and vector control (heatmap). FPKM, fragments per kilobase of transcript per million fragments mapped. *CLCN2* and *CYP11B2* show the largest increase in expression versus control. Genes involved in adrenal function or calcium pathway are highlighted. (b) Relative expression levels of *CYP11B2* (box, interquartile range; whiskers, 1.5x interquartile range; line, median) in the H295R cell line after transfection with empty vector (control), *CLCN2$^{WT}$* (blue), and *CLCN2$^{MUT}$* (yellow). Parallel transfections and real-time PCRs were performed in each group. *CYP11B2* expression significantly increases after transfection of *CLCN2$^{MUT}$*. (c) Resting membrane potential (plots as above) of HAC15 cells stably expressing *CLCN2* (WT or p.Arg172Gln) and untransfected controls. WT and p.Arg172Gln cause significant depolarization versus control, and p.Arg172Gln causes significant depolarization versus WT. (d) Model of ClC-2 function in human adrenal glomerulosa. Resting cells are hyperpolarized. Angiotensin II (AngII) and hyperkalemia cause depolarization, activation of voltage-dependent calcium channels, calcium influx, and increased *CYP11B2* expression via the transcription factor *NR4A2* (*NURR1*). ClC-$2^{MUT}$ causes increased *CYP11B2* expression by membrane depolarization via increased $Cl^-$ efflux. **, p<0.01; ***, p<0.001; ****, p<0.0001.

**SEQ ID NO: 1 CLCN2 Isoform 1 Amino Acid Sequence:**

MAAAAAEEGMEPRALQYEQTLMYGRYTQDLGAFAKEEAARIRLGGPEPWKGPPSSRAAPE
LLEYGRSRCARCRVCSVRCHKFLVSRVGEDWIFLVLLGLLMALVSWVMDYAIAACLQAQQ
WMSRGLNTSILLQYLAWVTYPVVLITFSAGFTQILAPQAVGSGIPEMKTILRGVVLKEYLTL
KTFIAKVIGLTCALGSGMPLGKEGPFVHIASMCAALLSKFLSLFGGIYENESRNTEMLAAAC
AVGVGCCFAAPIGGVLFSIEVTSTFFAVRNYWRGFFAATFSAFIFRVLAVWNRDEETITALF
KTRFRLDFPFDLQELPAFAVIGIASGFGGALFVYLNRKIVQVMRKQKTINRFLMRKRLLFPA
LVTLLISTLTFPPGFGQFMAGQLSQKETLVTLFDNRTWVRQGLVEELEPPSTSQAWNPPRA

NVFLTLVIFILMKFWMSALATTIPVPCGAFMPVFVIGAAFGRLVGESMAAWFPDGIHTDSS
TYRIVPGGYAVVGAAALAGAVTHTVSTAVIVFELTGQIAHILPVMIAVILANAVAQSLQPSLY
DSIIRIKKLPYLPELGWGRHQQYRVRVEDIMVRDVPHVALSCTFRDLRLALHRTKGRMLA
LVESPESMILLGSIERSQVVALLGAQLSPARRRQHMQERRATQTSPLSDQEGPPTPEASVCF
QVNTEDSAFPAARGETHKPLKPALKRGPSVTRNLGESPTGSAESAGIALRSLFCGSPPPEAA
SEKLESCEKRKLKRVRISLASDADLEGEMSPEEILEWEEQQLDEPVNFSDCKIDPAPFQLVE
RTSLHKTHTIFSLLGVDHAYVTSIGRLIGIVTLKELRKAIEGSVTAQGVKVRPPLASFRDSAT
SSSDTETTEVHALWGPHSRHGLPREGSPSDSDDKCQ

**SEQ ID NO: 2 *CLCN2* cDNA sequence:**

ATGGCGGCCGCGGCGGCGGAGGAAGGGATGGAGCCACGGGCGCTGCAGTACGAGCAGAC
CCTGATGTATGGCCGGTACACTCAGGACCTTGGGGCCTTTGCCAAAGAGGAAGCTGCTCG
GATTCGCCTGGGAGGGCCTGAACCCTGGAAAGGTCCCCCTTCCTCTCGGGCTGCCCCAGA
GCTCTTGGAATATGGACGGAGCCGTTGCGCCCGATGCCGCGTCTGTTCTGTCCGCTGCCA
CAAGTTCCTAGTATCCAGGGTTGGTGAAGATTGGATCTTCCTGGTCCTGCTGGGGCTTCTC
ATGGCATTGGTCAGCTGGGTCATGGACTATGCCATTGCTGCCTGTCTGCAAGCCCAGCAG
TGGATGTCCCGGGGCTTGAACACCAGCATCTTGCTCCAGTACCTGGCCTGGGTCACCTACC
CTGTTGTCCTCATCACTTTCTCAGCCGGATTCACACAGATCCTGGCCCCTCAGGCTGTCGG
CTCTGGCATCCCTGAGATGAAGACCATCTTGCGGGGAGTGGTGCTGAAAGAATACCTCAC
ACTCAAGACCTTTATAGCTAAGGTCATTGGGCTGACCTGCGCCCTAGGCAGCGGGATGCC
GCTTGGCAAAGAGGGCCCTTTTGTGCATATCGCAAGCATGTGTGCTGCCCTTCTCAGCAAG
TTCCTCTCCCTCTTTGGGGGTATCTATGAGAATGAATCCCGGAACACAGAGATGCTGGCTG
CCGCCTGTGCCGTGGGGGTGGGCTGCTGCTTCGCGGCACCTATTGGAGGCGTCCTCTTCA
GCATCGAGGTCACCTCCACCTTCTTTGCAGTGCGGAACTACTGGCGGGGCTTCTTCGCTGC
CACCTTCAGTGCCTTCATCTTCCGGGTCTTGGCAGTCTGGAACCGGGATGAAGAGACTATT
ACAGCCCTCTTCAAAACCCGATTCCGGCTCGACTTCCCCTTTGACCTGCAGGAGCTGCCAG
CCTTTGCTGTCATTGGTATTGCTAGTGGCTTCGGTGGAGCCCTCTTTGTCTACCTGAACCG
GAAGATTGTCCAGGTGATGCGGAAGCAGAAAACCATCAATCGCTTCCTCATGAGGAAACG
CCTGCTCTTCCCGGCTCTGGTGACCCTGCTCATCTCCACGCTGACCTTCCCCCCTGGCTTTG
GACAGTTCATGGCTGGACAGCTCTCACAGAAAGAGACGCTGGTCACCCTGTTTGACAATC
GGACGTGGGTCCGCCAGGGCCTGGTGGAGGAGCTAGAACCACCCAGCACCTCACAGGCCT
GGAACCCACCACGTGCCAACGTCTTCCTCACCCTGGTCATCTTCATTCTCATGAAGTTCTG
GATGTCTGCACTGGCCACCACCATCCCAGTTCCCTGTGGGGCCTTCATGCCTGTCTTTGTC
ATTGGAGCAGCATTTGGGCGTCTGGTGGGTGAAAGCATGGCTGCCTGGTTCCCAGATGGA
ATTCATACGGACAGCAGCACCTACCGGATTGTGCCTGGGGGCTACGCTGTGGTCGGGGCA
GCTGCGCTGGCAGGAGCGGTGACACACACAGTGTCCACGGCTGTGATCGTGTTCGAGCTC
ACAGGCCAGATTGCCCACATCCTGCCTGTCATGATCGCCGTCATCCTGGCCAACGCTGTCG
CCCAGAGTCTGCAGCCCTCCCTCTATGACAGCATCATCCGAATCAAGAAACTGCCCTACCT

GCCTGAGCTCGGCTGGGGCCGCCACCAGCAGTACCGGGTGCGTGTGGAGGACATCATGGT
GCGGGATGTTCCCCATGTGGCCCTCAGCTGCACCTTCCGGGACCTGCGTTTGGCACTGCAC
AGGACCAAGGGCCGAATGCTGGCCCTAGTGGAGTCCCCTGAGTCCATGATTCTGCTGGGC
TCCATCGAGCGTTCACAGGTGGTGGCATTGTTGGGGGCCCAGCTGAGCCCAGCCCGCCGG
CGGCAGCACATGCAGGAGCGCAGAGCCACCCAGACCTCTCCACTATCTGATCAGGAGGGT
CCCCCTACCCCTGAGGCTTCTGTCTGCTTCCAGGTGAACACAGAAGACTCAGCCTTCCCAG
CAGCCCGGGGGGAGACCCACAAGCCCCTAAAGCCTGCACTCAAGAGGGGGCCCAGTGTCA
CCAGGAACCTCGGAGAGAGTCCCACAGGGAGCGCAGAGTCGGCAGGCATCGCCCTCCGGA
GCCTCTTCTGTGGCAGTCCACCCCCTGAGGCTGCTTCGGAGAAGTTGGAATCCTGTGAGA
AGCGCAAGCTGAAGCGTGTCCGAATCTCCCTGGCAAGTGACGCGGACCTGGAAGGCGAGA
TGAGCCCTGAAGAGATTCTGGAGTGGGAGGAGCAGCAACTAGATGAACCTGTCAACTTCA
GTGACTGCAAAATTGATCCTGCTCCCTTCCAGCTGGTGGAGCGGACCTCTTTGCACAAGAC
TCACACTATCTTCTCACTGCTGGGAGTGGACCATGCTTATGTCACCAGTATTGGCAGACTC
ATTGGAATCGTTACTCTAAAGGAGCTCCGGAAGGCCATCGAGGGCTCTGTCACAGCACAG
GGTGTGAAAGTCCGGCCGCCCCTCGCCAGCTTCCGAGACAGTGCCACCAGCAGCAGTGAC
ACGGAGACCACTGAGGTGCATGCACTCTGGGGGCCCCACTCCCGTCATGGCCTCCCCCGG
GAGGGCAGCCCTTCCGACAGCGACGACAAATGCCAATGA

EXAMPLES

In context of the present invention an additional affected individual of a kindred compared to previous studies ((Stowasser, M. et al. Clin Exp Pharmacol Physiol 19, 319-22 (1992)) was recruited (family 3, Fig. 1, Table 1). The inventors performed exome sequencing of three affected subjects, revealing two shared novel protein-changing heterozygous variants in *CLCN2* (p.Arg172Gln, NP_004357) and *LINGO1* (p.His591Gln, NP_116197). *CLCN2* was considered the more likely candidate gene based on conservation (Fig. 1), expression levels in human adrenal cortex (8.14 for CLCN2, 5.91 for LINGO1, log2 scale, mean expression of all genes 7.206), and segregation analysis in the pedigree (Fig. 1).

**Table 1. Clinical Characteristics of Subjects with *CLCN2* Variants**

| Family ID | Subject ID | *CLCN2* variant | Gender | Aged dx / blood draw | BP (mmHg) | BP %ile | $K^+$ (mM) | Aldo (ng/dl) | PRA (ng/ml /h) | ARR (ng/dl: ng/ml/h) |
|---|---|---|---|---|---|---|---|---|---|---|
| Normal range | | | | | <140/90 in adults | <95th in children | 3.5-5.5 | | | < 20 |
| 3 | 2 | p.Arg172Gln | F | 24 | 120/80 on 3 meds | NA | 3.0 | 40.2 | 0.3 | 134.0 |
| 3 | 4 | p.Arg172Gln | M | 36 | 98/70 (age 24) | NA | 4.2 | 24.5 | 1.2 | 20.4 |
| 3 | 5 | p.Arg172Gln | F | 19 | 150/100 on 1 med | NA | 3.3 | 27.4 | 0.1 | 274.0 |
| 3 | 7 | p.Arg172Gln | F | 20 | 140/100 | NA | 3.8 | 26.5 | 0.1 | 265.0 |
| 3 | 9 | p.Arg172Gln | M | 18 | 120/80 (age 19) | NA | 4.0 | 24.7 | 1.5 | 16.5 |
| 3 | 10 | p.Arg172Gln | M | 17 | 130/85 | 90-95th | 4.0 | 26.2 | 0.1 | 262.0 |
| 3 | 11 | p.Arg172Gln | F | 14 | 94/62 | ≤ 50th | 4.0 | 21.2 | 0.1 | 212.0 |
| 3 | 35 | p.Arg172Gln | F | 16 | 170/110 | >99th | 3.4 | 25.5 | ≤0.2 | ≥127.5 |
| 1786 | 1 | p.Arg172Gln | F | 15 | 150/100 | >99th | 2.9 | 47.9 | <1.0 | >47.9 |
| 1786 | 2 | p.Arg172Gln | F | 32 | 118/68 on 2 meds | NA | 3.0 | 46.3 | <1.0 | >46.3 |
| 1786 | 3 | p.Arg172Gln | M | 13 | 121/75 (24 h) | >75th | 4.4 | 12.0 | <0.6 | >20.0 |
| 537 | 1 | p.Arg172Gln | F | 11 | 160/120 | >99th | 3.0 | 26.0 | 0.3 | 86.7 |
| 318 | 1 | p.Arg172Gln | M | 7 | 170/140 | >99th | 2.6 | 9.5 | 0.21 | 45.2 |
| 1281 | 1 | p.Met22Lys | F | 1 | 117/71 | >99th | 4.1 | 17.0 | <0.5 | >34.0 |
| 531 | 1 | p.Tyr26Asn | F | 6 | 280/188 at age 20 | NA | NA | 100.0 | <3.0 | >33.3 |
| 840 | 1 | p.Ser865Arg | M | 15 | 130/100 | 90-95th/>9 9th | 3.2 | 37.0 | 0.2 | 185.0 |
| 1492 | 1 | p.Lys362del | M | 0.2 | 150/90 | >99th | 4.0 | 63.8 | <0.15 | >425.3 |

Age dx, age at diagnosis; BP, blood pressure (office unless otherwise indicated); BP %ile, blood pressure percentile for subjects under age 18 years; $K^+$, serum potassium level; Aldo, serum aldosterone level; PRA, plasma renin activity; ARR, aldosterone/renin ratio. F, female; M, male; NA, not available or not applicable; med(s), antihypertensive medication(s). Reference values are given below each parameter.

The inventors next searched for rare (allele frequency <$10^{-5}$ in public databases) damaging variants in *CLCN2* and *LINGO1* among the exomes of 35 unrelated individuals diagnosed with unsolved primary aldosteronism by age 10 years - an extreme phenotype. Only *CLCN2* showed such variants. All four were heterozygous and absent in public databases. Remarkably, one proband had the identical p.Arg172Gln variant found in family 3. In three additional subjects, p.Met22Lys and p.Tyr26Asn occurred at positions conserved through invertebrates, and one variant produced a new splice donor site resulting in an in-frame deletion (p.Lys362del) (Fig. 1, see below).

Analysis of *CLCN2* in 45 additional unrelated subjects diagnosed with primary aldosteronism by age 20 years revealed two additional occurrences of p.Arg172Gln (Fig. 1). Additionally, p.Ser865Arg occurred at a moderately conserved position (Fig. 1). Sanger sequencing confirmed all variants. Sequencing of Arg172 in 1587 additional subjects referred for potential Mendelian hypertension, including 375 with primary aldosteronism diagnosed after age 20, identified no p.Arg172Gln variants, supporting enrichment in early-onset primary aldosteronism.

Among the four kindreds with p.Arg172Gln, one mutation (in kindred 318) occurred *de novo* (absent in proband's biological parents; Fig. 1). Among the other three kindreds, the maximum lengths of shared mutation-linked haplotypes between pairs of individuals ranged from 4,894 bp to 357,885 bp, with the putative last shared ancestor occurring ~651 (95% CI, 203-2615) to -50,000 generations ago (95% CI, 10,000-infinity). While extremely remote common ancestry is a possibility, independent occurrence is overwhelmingly likely. After finding this mutation in the first family, the probability of finding, by chance, three additional independent instances of p.Arg172Gln (one de novo), among 80 probands is $6.5 \times 10^{-12}$ (see Methods). The probability of any pair of these variants being identical by descent from a remote common ancestor is even lower. Lastly, the burden of rare protein-altering *CLCN2* variants in primary aldosteronism kindreds is significantly higher than in controls (8/81 vs. 6/3578, $p = 1.3 \times 10^{-10}$, relative risk 58.9).

Sanger sequencing identified eight carriers of the p.Arg172Gln variant in family 3 (Fig. 1, Table 1). Seven carriers had elevated aldosterone/renin ratios (ARRs, a screening test for primary aldosteronism); those tested had positive confirmatory fludrocortisone suppression tests (FSTs) and non-lateralizing aldosterone production. One subject had repeatedly normal ARRs, suggesting incomplete penetrance. Subject 3-1, diagnosed with hypertension in her 30s and with primary aldosteronism at age 66 years, was *CLCN2* wildtype. In addition to later onset, she was distinct in having increased aldosterone with upright posture, typical of sporadic idiopathic hyperaldosteronism5. In kindred 1786, the proband's affected mother and brother carried the p.Arg172Gln variant. The brother had borderline ARR with suppressed renin and prehypertension at age 13 years. Subject 1492-1 was diagnosed with hypertension and primary aldosteronism in infancy, but became normotensive by age 2, suggesting variable expressivity with age. Among other probands, the p.Met22Lys variant was *de novo.* Thus in two of the four kindreds with parental data, rare *CLCN2* mutations were *de novo.*

In silico splice site analysis of the variant in kindred 1492 predicted the creation of a new splice donor at the end of exon 10, three base pairs upstream of the normal splice donor. In a splicing assay in HEK cells, the wildtype exon was normally spliced, but the mutation resulted exclusively in splicing at the predicted upstream site, producing an in-frame deletion of codon 362.

ClC-2, the chloride channel encoded by *CLCN2*, is found in many tissues, including brain, kidney, lung, and intestine. Additionally, *CLCN2* RNA is found in the adrenal gland (see above). Immunohistochemistry with an antibody specific for ClC-2 revealed intense staining of human adrenal zona glomerulosa (Fig. 2), consistent with a role in regulating aldosterone production.

Chloride channels can conduct excitatory (membrane depolarizing) chloride efflux or inhibitory influx, depending on the ratio of chloride concentration on either side of the cell membrane. The intracellular chloride concentration ($[Cl^-]_{int}$) in mouse adrenal gland slices (Fig. 3a,b) was determined using fluorescence lifetime imaging microscopy; the method is based on the concentration-dependent fluorescence quenching of a chloride-sensitive dye. The median value of $[Cl^-]_{int}$ was 74.7 mM. The distribution of intracellular glomerulosa chloride concentrations is rather broad, as expected for a dynamic equilibrium in cells with oscillating membrane potentials. With a normal plasma $Cl^-$ concentration of 100 mM, the calculated chloride reversal potential at 37°C is -8 mV, predicting that opening of ClC-2 in glomerulosa cells will result in chloride efflux and depolarization from the resting potential of about -80 mV. This depolarization is predicted to activate voltage-gated $Ca^{2+}$ channels, inducing aldosterone biosynthesis.

A facultative subunit of ClC-2 in glia, GlialCAM/HEPACAM23, is not expressed in human adrenal gland (GTEx portal, see URLs). The inventors therefore expressed wildtype ($ClC\text{-}2^{WT}$) and each of the mutant ClC-2s ($ClC\text{-}2^{MUT}$) alone in HEK293T cells and performed whole-cell patch clamp electrophysiology at $[Cl^-]_{int}$=75 mM. $ClC\text{-}2^{WT}$ channels are closed at depolarized voltages and activate slowly at voltages negative to the chloride reversal potential. All mutants shifted the activation curve to more positive voltages (Fig. 3e). CLC channels are homodimers, with each subunit forming a separate conduction pathway. Each protopore can be individually opened and closed by a fast protopore gate, and a common slow gating mechanism acts on both pores. Whereas p.Ser865Arg altered protopore open probability and deactivation time constants, all other mutations modified common gating by increasing the minimum open probability and accelerating activation and deactivation time constants. Mass spectrometry demonstrated that p.Ser865 was phosphorylated, suggesting a regulatory mechanism. The observed gating alterations with $ClC\text{-}2^{MUT}$ predict significantly larger chloride efflux versus ClC-2[WT] in glomerulosa cells at physiological membrane potentials.

To characterize the impact of ClC-2[WT] and ClC-2[MUT] in human adrenal glomerulosa cells, the inventors expressed channels in human H295R adrenocortical cancer cells, an established model of aldosterone production. RNA-Seq (Fig. 4a) demonstrated that transfection of untagged WT and p.Arg172Gln *CLCN2* both significantly increased expression of *CYP11B2* and its upstream regulator *NR4A2* (*NURR1*); *RGS4*, which provides feedback inhibition of Ang II-triggered signaling, was also upregulated. Quantitative real-time PCR of *CYP11B2* revealed that transfection of CLCN2MUT produced significantly greater increases in *CYP11B2* expression than *CLCN2*WT (Fig. 4b). In contrast, transfection of loss-of-function *CLCN2* mutations did not change *CYP11B2* expression. H295R cells and their subclone HAC15 have negative membrane potentials. Current-clamp recordings demonstrated significant depolarization of HAC15 upon WT *CLCN2* expression; p.Arg172Gln amplified this effect (Fig. 4c).

The finding of four independent occurrences of p.Arg172Gln (one *de novo*), along with four additional novel variants (one *de novo*) among 81 probands with early-onset primary aldosteronism provides strong evidence implicating these variants in disease pathogenesis. The localization of ClC-2 in adrenal zona glomerulosa is consistent with this observation. The electrophysiologic impact of mutant channels and their effect on aldosterone synthase expression demonstrate that these mutations cause gain of function, producing membrane depolarization and increasing CYP11B2 expression (Fig. 4d).

Retrospectively, efforts to map the disease gene in family 3 were challenged by a phenocopy (sporadic idiopathic hyperaldosteronism) in subject 3-1, incomplete penetrance and phenotypic uncertainty. Rare *CLCN2* variants explained primary aldosteronism in ~10% of the early-onset cohort studied, suggesting there are likely additional genes yet undiscovered. Genetic testing for germline mutations in *CLCN2* and other early primary aldosteronism genes can be useful for establishing diagnosis, defining treatment options and assessing risk to future offspring.

Probands with FH-II showed early-onset primary aldosteronism and hypertension, often with hypokalemia. Hypertension was controlled with mineralocorticoid receptor antagonists or other antihypertensives. This phenotype appears indistinguishable from patients with *CACNA1H* mutations. Hybrid steroid production and/or response to glucocorticoids, historically used to diagnose GRA, were absent, as were massive adrenal hyperplasia (present in many subjects with *KCNJ5* variants) and neurodevelopmental abnormalities (characteristic of *CACNA1D* mutation). Despite widespread *CLCN2* expression, subjects with gain-of-function *CLCN2* variants shared no apparent pathology other than primary aldosteronism, whereas loss-of-function *CLCN2* variants cause leukoencephalopathy with ataxia, with a similar phenotype in mice. Incomplete penetrance or phenotypic amelioration with age, as sometimes occurs with germline mutations in *CYP11B2, KCNJ5, CACNA1D* and *CACNA1H,* occurred in some subjects with *CLCN2* mutations.

The findings of the invention for the first time implicate activity of an anion channel in the regulation of aldosterone biosynthesis, primary aldosteronism and hypertension.

**Materials and Methods**

Study subjects included the Australian kindred[2,32,37], 35 unrelated primary aldosteronism subjects without known disease-causing mutations, diagnosed by age 10 years (clinical characteristics published[13]), and 45 subjects diagnosed with primary aldosteronism by age 20 years. Selected families had additional members recruited. Controls were 3,578 unaffected parents of autistic offspring[38]. Research protocols were approved by local institutional review boards at Yale University and University of Queensland, and all probands and family members provided informed consent. Primary aldosteronism was diagnosed based on elevated aldosterone/renin ratio (ARR, >20 ng/dl:ng/ml/h or equivalent values)[5], with aldosterone >15 ng/dl, or marginally elevated values in the presence of hypokalemia. Confirmatory testing was performed according to the referring centers' guidelines[5]. Venous blood or saliva samples were obtained from subjects and family members and subjected to exome and/or Sanger sequencing[13].

**DNA preparation and exome sequencing.** DNA was prepared from venous blood or saliva samples using standard procedures. Exome capture was performed using the 2.1M NimbleGen Exome reagent (Roche NimbleGen, Madison, WI), and 75 base paired end sequencing on the Illumina (San Diego, CA) platform and analysis were performed as described[13].

**Sanger sequencing of genomic DNA and genotyping of parent-offspring trios.** Direct bidirectional Sanger sequencing of candidate variants from genomic DNA of indicated subjects was performed at Beckman Coulter Genomics (UK) or the Keck DNA sequencing facility at Yale University following PCR amplification. Rare variants identified in index cases through exome sequencing were genotyped by targeted PCR and Sanger sequencing in both parents to confirm paternity / maternity.

**Immunohistochemistry and immunofluorescence.** Formalin-fixed, paraffin-embedded 5 □m human adrenal gland sections were obtained from US Biomax (Rockville, MD, USA) and Pantomics (Richmond, CA, USA). Immunohistochemistry was performed as described[7], with the exception that 10% donkey serum was used for blocking. The concentration of the antigenic peptide was 0.4 mg/ml, and 1 μg peptide per μg antibody was used. Images were recorded on a Zeiss (Oberkochen, Germany) Axioplan 2 Imaging microscope (10x and 40x objectives) with a Zeiss AxioCam Mrc5 camera. Image cropping was performed in Adobe Illustrator CS4. Primary antibody against ClC-2 was HPA014545 (Sigma-Aldrich Prestige Antibodies, St. Louis, MO, USA; 1:100, incubation overnight at 4°C), and antibody against Dab2 was #sc-13982 (Santa Cruz, Santa Cruz, CA, USA; 1:100 dilution). Secondary antibody was donkey α-rabbit (#035-152, 1:200, Jackson, Bar Harbor, ME, USA, 2 h at room temperature) for human samples. To confirm the selection of the zona glomerulosa in mouse adrenal slices for fluorescence lifetime imaging (FLIM), slices were stained with the Dab2 antibody (1:100 dilution, incubation overnight at 4°C). Secondary antibody was donkey α-rabbit conjugated to Alexa Fluor 647 (A-31573, ThermoFisher Scientific; 1:1000, 2h at room temperature). Immunofluorescent images were recorded on a Leica TCS SP5 laser scanning confocal microscope (Leica Microsystems, Heidelberg, Germany).

**Molecular cloning.** Site directed mutagenesis (QuikChange, Agilent Technologies, Santa Clara, CA) was performed to introduce mutations into pcDNA5/FRT/TO ClC-2[24] according to the manufacturer's instructions. Each construct was validated by sequencing of the entire coding region. Mutant cDNAs were subcloned in frame into the pRcCMV vector containing the YFP cDNA using *NotI* and *PmlI* for use in confocal microscopy only. Two independent clones were assessed in all experiments.

**Generation of stable cell lines.** Stable HEK293 cell lines were generated using the Flp-In T-REx system (Invitrogen, Life Technologies, Carlsbad, CA, USA) according to the manufacturer's instructions. HEK293 cells do not express HEPACAM (Human Protein Atlas, see URLs). Flp-In T-REx 293 cells (authenticated, Eurofins Genomics) were cultured in high glucose DMEM (Biochrom GmbH, Berlin, Germany) with 10% FBS (Biochrom), 1% Penicillin/Streptomycin, 100 μg/mL Zeocin and 15μg/mL Blasticidin (all Invitrogen) at 37°C and 5% $CO_2$ humidified atmosphere. Cells were transfected with 2.4 μg pcDNA5/FRT/TO + insert (*CLCN2* mutants: p.Met22Lys, p.Tyr26Asn, p.Arg172Gln, p.Lys362del, p.Ser865Arg; two clones each) and 21.6 μg pOG44 using Lipofectamine 2000 (Invitrogen) and OPTIMEM (Gibco by Life Technologies, Carlsbad, CA, USA). The following day, the medium was changed to high glucose DMEM + 10% Tet-free FBS (Gibco) and 1% Penicillin/Streptomycin. 48 h after transfection, cells were split onto 15 cm dishes and selected with 15 μg/mL Blasticidin and 150 μg/mL Hygromycin (Invitrogen). Single colonies were selected. Variants were confirmed by Sanger sequencing of DNA extracted from stable cell lines, and inducible expression was verified by western blot (ClC-2 antibody #ACL-002, Alomone labs, Jerusalem, Israel).

The HAC15 cell line was kindly provided by Dr. William Rainey (University of Michigan), authenticated by short tandem repeat (STR) analysis (ATCC Cell Line Authentication Service) and cultured in DMEM/F12 (GlutaMAX; Gibco) + 5% HyClone Cosmic Calf Serum (CCS; GE Healthcare Life Sciences, Buckinghamshire, UK) +1% Penicillin-Streptomycin (Gibco) + 1% Insulin-Transferrin-Selenium (ITS; Gibco) +1% MEM Non-Essential Amino Acids Solution (Gibco) + 0.1% CD Lipid Concentrate (Gibco) at 37°C and 5% $CO_2$ in humidified atmosphere. Stable cell lines were prepared using the Piggybac transposon system (System Biosciences, Palo Alto, CA, USA). cDNAs of *CLCN2* (WT and Arg172Gln) were subcloned into pENTR-2B-Dual using NotI and XhoI. Gateway LR recombination (Invitrogen) was performed with pPiggybac-EF1 Neo + pTF rLTA. Inserts were verified by Sanger sequencing. HAC15 cells were transfected using an Amaxa Nucleofector I (Lonza, Cologne, Germany; 2 million cells, 2 μg plasmid DNA, 0.8 μg Super Piggybac transposase; program X-005). After 48h, selection was initiated by addition of 5 μg/mL Blasticidin (Gibco) to the growth medium. Inducible expression was verified by western blot (ClC-2 antibody #ACL-002) after incubation with 1 μg/mL Doxycycline (Sigma Aldrich) for 24h.

**Preparation of acute adrenal slices.** After anesthetizing animals with isoflurane and decapitation, both adrenal glands were rapidly removed and placed in ice-cold Bicarbonate Buffered Saline (BBS) (125 mM NaCl, 2 mM KCl, 26 mM $NaHCO_3$, 0.1 mM $CaCl_2$, 5 mM $MgCl_2$, 10 mM glucose, constantly oxygenated with 5% $CO_2$ in $O_2$) for the removal of surrounding fat. The adrenal glands were embedded in 4% agarose in BBS, mounted, cut at 4°C (150-200 μm thick) with a Microm HM 650V (Thermo Scientific, Walldorf, Germany; frequency 60 Hz, amplitude 1 mm, drive 10) and held at 35°C for 30 min in BBS. Slices were subsequently stored in BBS at 37°C for further experiments. During each experiment, slices were constantly perfused with solution at 37°C, and all measurements were completed within 8 h of organ removal.

**Fluorescence lifetime imaging microscopy (FLIM).** Prior to chloride imaging experiments, adrenal slices were incubated in BBS containing 10 mM 1-(ethoxycarbonylmethyl)-6-methoxyquinolinium bromide (MQAE; Sigma-Aldrich, Munich, Germany[39]) for 45-60 min at room temperature. Slices were transferred to an imaging chamber, perfused with BBS solution containing 2 mM instead of 0.1 mM $Ca^{2+}$, and FLIM was performed as described[21]. The solution was perfused through a heating coil resulting in a temperature of 37°C in the perfusion chamber. Fluorescence was stimulated by two-photon excitation ($\lambda_{exc}$ = 750 nm), MQAE fluorescence was filtered (short pass filter, 500 nm, $\lambda_{obs}$ < 510 nm; Omega Optical, Brattleboro, VT), and mean fluorescence lifetimes were measured using multidimensional time-correlated single photon counting (TCSPC). TCSPC electronics (SPC-152; Becker & Hickl) and acquisition software were used for FLIM as described[40]. We recorded data of 12 slices from 5 different C57BL/6 mice (2 male, 3 female) of age 3 months or older. For chloride concentration calibration, MQAE fluorescence lifetimes of preset [$Cl^-$] were measured. Adrenal slices were incubated in HEPES-buffered solution (140 mM $K^+$, 10 mM $Na^+$, 10 mM HEPES, 10-80 mM $Cl^-$, 70-140 mM gluconate, adjusted to 310 mOsmol/L with K-gluconate and to pH 7.4 with KOH, 37°C) containing 10 μM nigericin (sodium salt; Sigma-Aldrich, Munich, Germany) and 10 μM tributyltin (chloride salt; Sigma-Aldrich)[21,40-42]. The inverse fluorescence lifetime (1/τ) was plotted, and the calibration curve was fitted as described before[21]. The Stern-Volmer constant (Ksv = 3.96) was determined as the product of $\tau_o$ and the slope of the calibration curve. Since the fluorescence lifetime of MQAE is reduced by chloride via collisional quenching, MQAE fluorescence lifetimes and [$Cl^-$] show a linear relationship:

$$\frac{\tau_0}{\tau} = 1 + K_{SV}[Cl^-]$$

Zona glomerulosa $[Cl^-]_{int}$ could then be calculated according to this relationship.

The three outermost cell layers were assumed to form the zona glomerulosa based on their characteristic nucleus to cytoplasm ratio and the corresponding staining with anti-DAB2 (#sc-13982, Santa Cruz) performed separately. Each cell was defined as a region of interest (ROI) with the exclusion of the nucleus, and fluorescence lifetimes were determined as mean values of the average fluorescence lifetimes of all pixels in a given ROI. Fluorescence lifetimes were calculated using SPCImage 5.6 (Becker&Hickl, Berlin, Germany) and exported for further extraction in Fiji. Statistics were performed using SigmaPlot 12 (Systat) and Python 3.5.2 + numpy 1.12.1 + scipy 0.18.1 + pandas 0.19.2 + seaborn 0.7.1 using built-in functions. The FLIM datasets generated during or analyzed during the current study are available on request. Python scripts for analysis are based on built-in functions of the above mentioned packages but are available on request.

**Electrophysiological recordings.** Flp-In T-REx stable cell lines were used for electrophysiological recordings. For each construct, at least two clones using at least two separate preparations were included in the analysis. To avoid chloride depletion at large current amplitudes[24], Flp-In T-REx cells were used without induction. Whole-cell patch clamp currents were recorded on an EPC10 amplifier using PatchMaster software (both HEKA, Lambrecht/Pfalz, Germany). Borosilicate glass pipettes with open resistances between 0.9 and 2.5 MΩ were used. The extracellular solution for whole-cell recordings contained (in mM): NaCl (140), KCl (4), $CaCl_2$ (2), $MgCl_2$ (1) and HEPES (10) at pH 7.4. The intracellular solution contained (in mM): NaCl (73), $MgCl_2$ (1), NaGluconate (42), EGTA (5), HEPES (10) and MgATP (1) at pH 7.4. The liquid junction potential was calculated to be 1.9 mV and corrected for *a priori.* Cells were held at the calculated chloride reversal potential of -17.5 mV at rest.

Instantaneous current amplitudes at the fixed tail step to +60 mV were normalized and plotted against the preceding voltage step to reveal relative open probability curves. The durations of the voltage steps were 5 s for $ClC\text{-}2^{WT}$ and $ClC\text{-}2^{Ser865Arg}$ and 1 s for all other mutations so that steady-state open probabilities were determined. Open probabilities ($P_{open}$) were fitted using a modified Boltzmann equation:

$$P_{open}(V) = \frac{1}{1 + e^{\frac{-(V - V_{1/2})}{k}}}$$

to allow for a comparison of the half-maximal activation($V_{1/2}$).

Fitting of the activation and deactivation current traces with the sum of two exponential functions revealed fast ($\tau_1$) and slow ($\tau_2$) time constants, respectively:

$$I(t) = A_0 + A_1 \cdot e^{-\frac{t}{\tau_1}} + A_2 \cdot e^{\frac{-t}{\tau_2}}$$

CLC chloride channels are double-barreled channels with two conduction pathways. Protopores can be individually opened and closed by a fast gating process, but also jointly by slow common gating. Under the assumption that protopore and common gating processes are independent, the overall open probability equals the product of the respective individual open probabilities[43]:

$$P_{open} = P_{fast} \cdot P_{slow}$$

By inserting a 15 ms pulse to -220 mV between the variable test pulse and the tail pulse, the ClC-2 fast gate is maximally opened ($P_{fast}$ = 1)[44], and the common gate open probability can be determined. Fast protopore gate open probabilities were calculated by dividing the overall open probability by the common gate open probability.

Resting potentials in untransfected or in induced (1 µg tetracycline/ml medium for 24h) stably transfected HAC15 cells were measured using the perforated patch technique[45] in the current clamp mode. The extracellular solution contained (in mM): 140 NaCl, 10 HEPES, 4 KCl, 2 $CaCl_2$, 1 $MgCl_2$ adjusted to a pH of 7.4. The pipette solution contained (in mM): 130 KCl, 10 HEPES, 5 NaCl with the pH adjusted to 7.4. To maintain the native intracellular [$Cl^-$], we included the pore-forming, monovalent cation selective antibiotic gramicidin D (Sigma-Aldrich) in the pipette solution to obtain access to the inside of the cell[45]. Gramicidin stock solution (50 mg/ml in DMSO) was prepared daily, and the diluted solution (final concentration: 100 μg/ml) was prepared every 2 h. The tip of the pipette (open resistance 1.5-3 MΩ) was filled with solution lacking gramicidin to facilitate gigaseal formation. Break-in was typically observed after 15-45 minutes. Resting potentials were determined using a HEKA EPC-10 patch clamp amplifier and the PatchMaster software (HEKA Elektronik) from the mean of 10-60 s long voltage recording segments with the current clamped to 0 pA. Only cells that exhibited ClC-2 like currents (visible slow activation upon hyperpolarization and a current larger than 40 pA at -160 mV in subsequent voltage-clamp experiments) were used for analysis.

Analysis of all electrophysiological experiments was performed using FitMaster software (HEKA Elektronik), SigmaPlot 12 (Systat) and Python 3.5.2 + numpy 1.12.1 + scipy 0.18.1 + pandas 0.19.2 using built-in functions. The electrophysiology datasets generated during or analyzed during the current study are available on request. Python scripts for analysis are based on built-in functions of the above mentioned packages but are available on request. Normality was assessed by Shapiro-Wilk test.

**Culture of H295R cells.** H295R human adrenocortical cells (a kind gift of Dr. Matthias Haase, Düsseldorf) were authenticated by short tandem repeat (STR) analysis (ATCC Cell Line Authentication Service) and cultured in DMEM/F12 + HEPES (Gibco) + 2.5 % Ultroser G (Pall, Port Washington, NY, USA) + 1% Insulin-Transferrin-Selenium+ (ITS+; Corning, Corning, NY, USA) + 1 % Penicillin/Streptomycin (Gibco) at 37°C and 5% $CO_2$ in humidified atmosphere.

**Quantitative real-time PCR.** Three million H295R cells were resuspended in 100 μl Nucleofector solution R (Lonza) plus 3 μg plasmid DNA (pcDNA/FRT/TO empty vector, WT or mutant *CLCN2*) and electroporated with program P-20 using an Amaxa Nucleofector I (Lonza). After recovering of the cells in RPMI 1640 medium (Gibco) for 15 min at 37°C, they were plated on 12-well plates. 24h after transfection, H295R cells were starved in DMEM/F12 + HEPES + 0.1 % Ultroser G + 1 % Penicillin/Streptomycin for additional 24h. Total RNA was isolated using the RNeasy® Mini Kit (Qiagen GmbH, Hilden, Germany) and quantified with a Nanodrop 2000 (Thermo Scientific, Wilmington, DE, USA). After reverse transcription of RNA using Quantitect RT Kit (Qiagen), Taqman gene expression assays (Applied Biosystems, Foster City, CA, USA) for *GAPDH* (HS02758991_g1) as housekeeping gene and *CYP11B2* (Hs01597732_m1) as gene of interest were performed using the Taqman Gene Expression Master Mix. Each variant was assessed in parallel with wildtype and empty vector control. Gene expression was evaluated relative to the housekeeping gene and expressed as $2^{\Delta\Delta Ct}$. Normality was assessed by Shapiro-Wilk test. Statistical differences were assessed by ratio-paired two-tailed t tests (for normally distributed individual data), one-way ANOVA (for normally distributed multiple comparisons; adjusted p value reported) or Friedman test (multiple comparisons, no normal distribution; adjusted p value reported) in Graphpad Prism 7.

**Whole-transcriptome sequencing, read alignment and differential gene expression analysis.** H295R cells were transfected in two independent reactions as above, RNA was isolated using Trizol (Thermo Fisher Scientific), followed by DNase digest and column purification (RNeasy, Qiagen). Libraries were prepared after Poly A selection. Samples were sequenced on the Illumina HiSeq2500 at the Yale Center for Genome Analysis, producing a mean of 47.9 million 75-bp single-end reads. The quality of the raw sequencing reads was evaluated using FastQC version 0.10.4 (see URLs), and the base of lower quality (base quality score < 20) in the last position was trimmed. Tophat v2.1.1 was used to align the high quality sequencing reads to the reference human genome sequence build hg19. Differentially expressed genes were identified by Cuffdiff v2.2.1[47]. An FDR adjusted p value (q value) ≤ 0.05 and $|\log_2(\text{Fold Change})| \geq 1$ were set as the cutoffs for significantly differential expression.

**Estimation of the probability of observing one *de novo* and two transmitted p.Arg172Gln variants in *CLCN2*.** From the identification of the novel p.Arg172Gln variant in family 3, we calculated the probability of finding an identical *de novo* mutation and two independent instances of the same transmitted variant by chance among 80 probands. Using the genome-wide mutation rate of $1.67\times10^{-8}$ per base per generation from a recent study, we estimated that the probability of seeing any specific *de novo* mutation in one individual is $3.34\times10^{-8}$. For transmitted events, we applied the UnseenEst[49] algorithm to estimate the probability of finding an unseen missense mutation in human populations. A total of 33,778 healthy individuals were selected from the ExAC database[50] to match the population distribution of the 2,010 U.S. Census. The U.S. Census-matched dataset was trained in the UnseenEst[49] algorithm to estimate the frequency distribution of distinct unseen missense mutations for the US population. The predicted frequency distribution was used to extrapolate the probability of observing one unseen transmitted event (probability = $2.81\times10^{-5}$). Taken together, the probability of observing one *de novo* and two transmitted p.Arg172Gln mutations among 80 independent samples is estimated to be

$$\binom{80}{2} \times (2.81\text{x}10^{-5})^2 \times (1 - 2.81\text{x}10^{-5})^{78} \times \binom{78}{1} \times (3.34\text{x}10^{-8})^1 \times (1 - 3.34\text{x}10^{-8})^{77}$$ = $6.49\times10^{-12}$.

**Shared haplotypes and estimation of the mutation age of p.Arg172Gln in *CLCN2.*** Genotypes of SNPs flanking the *CLCN2*$^{Arg172Gln}$ mutation were extracted from exome data. To estimate the mutation age of the *CLCN2* p.Arg172Gln mutation testing the assumption that the mutation is identical by descent among each possible pair of kindreds with the variant (except the documented *de novo* mutation), we used the ESTIAGE algorithm to estimate the pairwise time of coalescence for the three pairs of kindreds as previously described[13,18]. ESTIAGE uses a maximum-likelihood approach to estimate the mutation age, which takes into account the frequencies of the shared allele at each marker and the recombination fractions between the mutation of interest and polymorphic markers located within or at the boundaries of the shared haplotype. Seventeen polymorphic markers spanning the shared haplotype were used for input. The marker allele frequencies were estimated from the Finnish and Non-Finnish European populations in the ExAC database[50], and the mutation rate was set to $2\times10^{-8}$.

**Data availability.** *CLCN2* variants are deposited in ClinVar (accession numbers SCV000606833-7), RNAseq data are at GEO (accession number GSE107030, see URLs).

**Code availability.** Code for gene burden analysis is available at github (see URLs).

**URLs:**

ClinVar:

https://www.ncbi.nlm.nih.gov/clinvar/;
github:
https://github.com/murim76/gene burden test/blob/master/Compare VCF for Scholl. pl

GEO series accession:

https://www.ncbi.nlm.nih.gov/geo/query/acc.cgi?acc=GSE107030;

The Human Protein Atlas:

http://www.proteinatlas.org/ENSG00000165478-HEPACAM/cell/HEK+293,
FastQC:
http://www.bioinformatics.bbsrc.ac.uk/projects/fastqc/
GTEx Portal:
http://www.gtexportal.org/home/gene/HEPACAM

**References**

1. Monticone, S. et al. Prevalence and Clinical Manifestations of Primary Aldosteronism Encountered in Primary Care Practice. J Am Coll Cardiol 69, 1811-1820 (2017).
2. Stowasser, M. et al. Familial hyperaldosteronism type II: five families with a new variety of primary aldosteronism. Clin Exp Pharmacol Physiol 19, 319-22 (1992).
3. Collaboration, N.C.D.R.F. Worldwide trends in blood pressure from 1975 to 2015: a pooled analysis of 1479 population-based measurement studies with 19.1 million participants. Lancet (2016).
4. Lim, S.S. et al. A comparative risk assessment of burden of disease and injury attributable to 67 risk factors and risk factor clusters in 21 regions, 1990-2010: a systematic analysis for the Global Burden of Disease Study 2010. Lancet 380, 2224-60 (2012).
5. Funder, J.W. et al. The Management of Primary Aldosteronism: Case Detection, Diagnosis, and Treatment: An Endocrine Society Clinical Practice Guideline. J Clin Endocrinol Metab 101, 1889-916 (2016).
6. Choi, M. et al. K+ channel mutations in adrenal aldosterone-producing adenomas and hereditary hypertension. Science 331, 768-72 (2011).
7. Scholl, U.I. et al. Somatic and germline CACNA1D calcium channel mutations in aldosterone-producing adenomas and primary aldosteronism. Nat Genet 45, 1050-4 (2013).
8. Azizan, E.A. et al. Somatic mutations in ATP1A1 and CACNA1D underlie a common subtype of adrenal hypertension. Nat Genet 45, 1055-60 (2013).
9. Beuschlein, F. et al. Somatic mutations in ATP1A1 and ATP2B3 lead to aldosterone-producing adenomas and secondary hypertension. Nat Genet 45, 440-4 (2013).
10. Fernandes-Rosa, F.L. et al. Genetic spectrum and clinical correlates of somatic mutations in aldosterone-producing adenoma. Hypertension 64, 354-61 (2014).
11. Lifton, R.P. et al. A chimaeric 11 beta-hydroxylase/aldosterone synthase gene causes glucocorticoid-remediable aldosteronism and human hypertension. Nature 355, 262-5 (1992).
12. Scholl, U.I. et al. Hypertension with or without adrenal hyperplasia due to different inherited mutations in the potassium channel KCNJ5. Proc Natl Acad Sci U S A 109, 2533-8 (2012).
13. Scholl, U.I. et al. Recurrent gain of function mutation in calcium channel CACNA1H causes early-onset hypertension with primary aldosteronism. Elife 4(2015).
14. Korah, H.E. & Scholl, U.I. An Update on Familial Hyperaldosteronism. Horm Metab Res 47, 941-6 (2015).
15. Spat, A. & Hunyady, L. Control of aldosterone secretion: a model for convergence in cellular signaling pathways. Physiol Rev 84, 489-539 (2004).
16. Carss, K.J., Stowasser, M., Gordon, R.D. & O'Shaughnessy, K.M. Further study of chromosome 7p22 to identify the molecular basis of familial hyperaldosteronism type II. J Hum Hypertens 25, 560-4 (2011).
17. Dong, C. et al. Comparison and integration of deleteriousness prediction methods for nonsynonymous SNVs in whole exome sequencing studies. Hum Mol Genet 24, 2125-37 (2015).
18. Genin, E., Tullio-Pelet, A., Begeot, F., Lyonnet, S. & Abel, L. Estimating the age of rare disease mutations: the example of Triple-A syndrome. J Med Genet 41, 445-9 (2004).
19. Dogan, R.I., Getoor, L., Wilbur, W.J. & Mount, S.M. SplicePort--an interactive splice-site analysis tool. Nucleic Acids Res 35, W285-91 (2007).
20. Thiemann, A., Grunder, S., Pusch, M. & Jentsch, T.J. A chloride channel widely expressed in epithelial and non-epithelial cells. Nature 356, 7-60 (1992).
21. Untiet, V. et al. Glutamate transporter-associated anion channels adjust intracellular chloride concentrations during glial maturation. Glia 65, 388-400 (2017).
22. Hu, C., Rusin, C.G., Tan, Z., Guagliardo, N.A. & Barrett, P.Q. Zona glomerulosa cells of the mouse adrenal cortex are intrinsic electrical oscillators. J Clin Invest 122, 2046-53 (2012).
23. Jeworutzki, E. et al. GlialCAM, a protein defective in a leukodystrophy, serves as a ClC-2 Cl(-) channel auxiliary subunit. Neuron 73, 951-61 (2012).
24. Stolting, G. et al. Regulation of ClC-2 gating by intracellular ATP. Pflugers Arch 465, 1423-37 (2013).
25. Niemeyer, M.I., Cid, L.P., Zuniga, L., Catalan, M. & Sepulveda, F.V. A conserved pore-lining glutamate as a voltage- and chloride-dependent gate in the ClC-2 chloride channel. J Physiol 553, 873-9 (2003).
26. Jentsch, T.J. Discovery of CLC transport proteins: cloning, structure, function and pathophysiology. J Physiol 593, 4091-109 (2015).
27. Rainey, W.E., Bird, I.M. & Mason, J.I. The NCI-H295 cell line: a pluripotent model for human adrenocortical studies. Mol Cell Endocrinol 100, 45-50 (1994).
28. Bassett, M.H., Suzuki, T., Sasano, H., White, P.C. & Rainey, W.E. The orphan nuclear receptors NURR1 and NGFIB regulate adrenal aldosterone production. Mol Endocrinol 18, 279-90 (2004).
29. Romero, D.G. et al. Regulators of G-protein signaling 4 in adrenal gland: localization, regulation, and role in aldosterone secretion. J Endocrinol 194, 429-40 (2007).
30. Garcia-Olivares, J. et al. Gating of human ClC-2 chloride channels and regulation by carboxy-terminal domains. J Physiol 586, 5325-36 (2008).
31. Tauber, P. et al. Cellular Pathophysiology of an Adrenal Adenoma-Associated Mutant of the Plasma Membrane Ca(2+)-ATPase ATP2B3. Endocrinology 157, 2489-99 (2016).
32. Lafferty, A.R. et al. A novel genetic locus for low renin hypertension: familial hyperaldosteronism type II maps to chromosome 7 (7p22). J Med Genet 37, 831-5 (2000).
33. Depienne, C. et al. Brain white matter oedema due to ClC-2 chloride channel deficiency: an observational analytical study. Lancet Neurol 12, 659-68 (2013).
34. Blanz, J. et al. Leukoencephalopathy upon disruption of the chloride channel ClC-2. J Neurosci 27, 6581-9 (2007).
35. Stowasser, M. et al. Clinical, biochemical and genetic approaches to the detection of familial hyperaldosteronism type I. J Hypertens 13, 1610-3 (1995).
36. Chorvatova, A., Gendron, L., Bilodeau, L., Gallo-Payet, N. & Payet, M.D. A Ras-dependent chloride current activated by adrenocorticotropin in rat adrenal zona glomerulosa cells. Endocrinology 141, 684-92 (2000).
37. Torpy, D.J. et al. Familial hyperaldosteronism type II: description of a large kindred and exclusion of the aldosterone synthase (CYP11B2) gene. J Clin Endocrinol Metab 83, 3214-8 (1998).

38. Krumm, N. et al. Excess of rare, inherited truncating mutations in autism. Nat Genet 47, 582-8 (2015).
39. Verkman, A.S. Development and biological applications of chloride-sensitive fluorescent indicators. Am J Physiol 259, C375-88 (1990).
40. Kaneko, H., Putzier, I., Frings, S., Kaupp, U.B. & Gensch, T. Chloride accumulation in mammalian olfactory sensory neurons. J Neurosci 24, 7931-8 (2004).
41. Bevensee, M.O., Apkon, M. & Boron, W.F. Intracellular pH regulation in cultured astrocytes from rat hippocampus. II. Electrogenic Na/HCO3 cotransport. J Gen Physiol 110, 467-83 (1997).
42. Chao, A.C., Dix, J.A., Sellers, M.C. & Verkman, A.S. Fluorescence measurement of chloride transport in monolayer cultured cells. Mechanisms of chloride transport in fibroblasts. Biophys J 56, 1071-81 (1989).
43. Accardi, A. & Pusch, M. Fast and slow gating relaxations in the muscle chloride channel CLC-1. J Gen Physiol 116, 433-44 (2000).
44. de Santiago, J.A., Nehrke, K. & Arreola, J. Quantitative analysis of the voltage-dependent gating of mouse parotid ClC-2 chloride channel. J Gen Physiol 126, 591-603 (2005).
45. Rhee, J.S., Ebihara, S. & Akaike, N. Gramicidin perforated patch-clamp technique reveals glycine-gated outward chloride current in dissociated nucleus solitarii neurons of the rat. J Neurophysiol 72, 1103-8 (1994).
46. Kim, D. et al. TopHat2: accurate alignment of transcriptomes in the presence of insertions, deletions and gene fusions. Genome Biol 14, R36 (2013).
47. Trapnell, C. et al. Differential analysis of gene regulation at transcript resolution with RNA-seq. Nat Biotechnol 31, 46-53 (2013).
48. Samocha, K.E. et al. A framework for the interpretation of de novo mutation in human disease. Nat Genet 46, 944-50 (2014).
49. Zou, J. et al. Quantifying unobserved protein-coding variants in human populations provides a roadmap for large-scale sequencing projects. Nat Commun 7, 13293 (2016).
50. Lek, M. et al. Analysis of protein-coding genetic variation in 60,706 humans. Nature 536, 285-91 (2016).

## Claims

1. An isolated nucleic acid molecule comprising a sequence encoding for chloride voltage-gated channel 2 (CLCN2) protein having the amino acid sequence shown in SEQ ID NO: 1, with at least one amino acid sequence variation compared to that sequence, or comprising a sequence according to SEQ ID NO: 2 with at least one nucleic acid sequence variation compared to that sequence.

2. The isolated nucleic acid molecule of claim 1, wherein the at least one amino acid sequence variation is at position Argl72, Met22, Tyr26, Ser865, and/or Lys362 in SEQ ID NO: 1.

3. The isolated nucleic acid molecule according to claim 1 or 2, wherein the at least one amino acid sequence variation is selected from Arg172Gln, Met22Lys, Tyr26Asn, Ser865Arg, and/or Lys362del.

4. An isolated nucleic acid primer or probe having a nucleic acid sequence identical to, or complementary to, or binding to under hybridizing conditions, a stretch of 8 to 20 consecutive nucleotides of the isolated nucleic acid molecule according to any one of claims 1 to 3, and wherein the stretch of 8 to 20 consecutive nucleotides comprises the codon sequence of the at least one amino acid sequence variation or nucleic acid sequence variation.

5. A method for detecting the presence of primary aldosteronism (PA) in a subject, the method comprising the steps of:

   a. Providing a biological sample of the subject, and
   b. detecting in the biological sample the presence or absence of one or more polymorphic sites in the CLCN2 gene or CLCN2 protein, and

   wherein the presence of the polymorphic site is indicative of the subject suffering from PA.

6. The method according to claim 5, wherein the one or more polymorphic site(s) in the *CLCN2* gene encode(s) for, or wherein the one or more polymorphic sites in the CLCN2 protein (ClC-2) is, at least one amino acid residue variation in the CLCN2 protein (SEQ ID NO: 1).

7. The method according to claim 6, wherein the at least one amino acid residue variation in the CLCN2 protein is at position Argl72, Met22, Tyr26, Ser865, and/or Lys362 in SEQ ID NO: 1.

8. The method according to any one of claims 5 or 7, wherein the PA is early onset PA and/or is familial hyperaldosteronism type II (FH-II).

9. The method according to claim any one of claims 5 to 8, wherein the step (b) comprises the detection of the binding of an antibody to the polymorphic site(s) in the CLCN2 protein, wherein said antibody is specific for to the polymorphic site(s) in the CLCN2 protein.

10. An isolated protein comprising an amino acid sequence encoded by a nucleic acid according to any one of claims 1 to 3.

11. An antigen binding protein specifically binding to an isolated protein according to claim 10.

12. The antigen binding protein according to claim 11, wherein the binding occurs at position Argl72, Met22, Tyr26, Ser865, and/or Lys362, of the protein.

13. A diagnostic kit, comprising an isolated nucleic acid according to claim 1 or 2, a primer or probe according to claim 3, an isolated protein according to claim 10, or an antigen binding protein according to any one of claims 11 or 12.

14. The diagnostic kit according to claim 13, for use in a method according to any one of claims 4 to 9.

15. Use of a diagnostic kit according to claim 13 in a method according to any one of claims 4 to 9.

Figure 1:

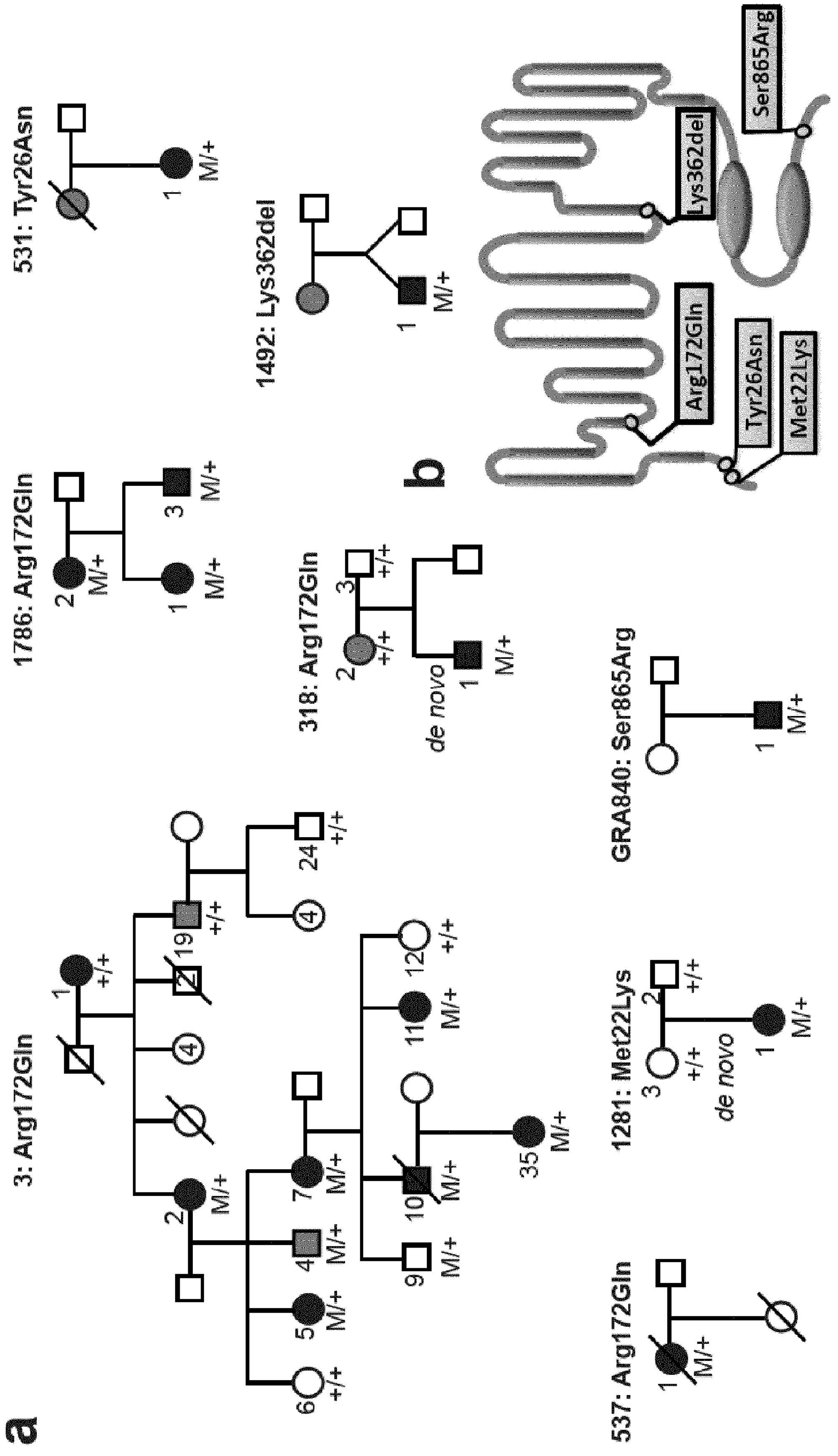
a
3: Arg172Gln
1786: Arg172Gln
531: Tyr26Asn
318: Arg172Gln
de novo
1492: Lys362del
GRA840: Ser865Arg
537: Arg172Gln
1281: Met22Lys
de novo
M/+
+/+
b
Arg172Gln
Tyr26Asn
Met22Lys
Lys362del
Ser865Arg

Figure 1 cont.:

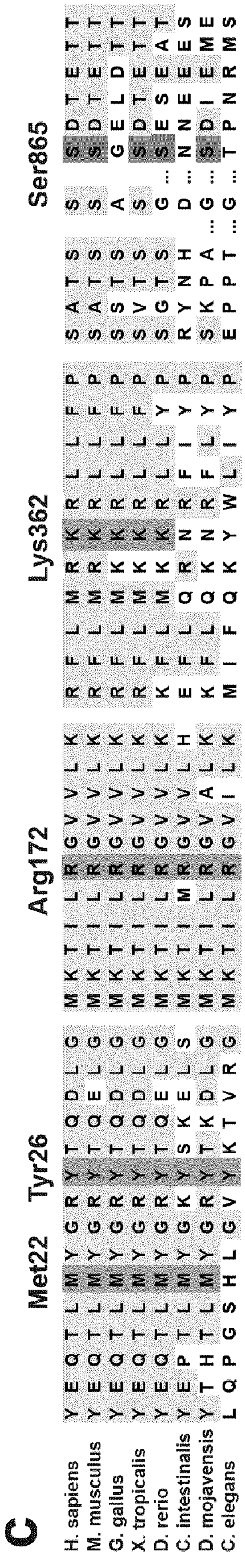

Figure 2:

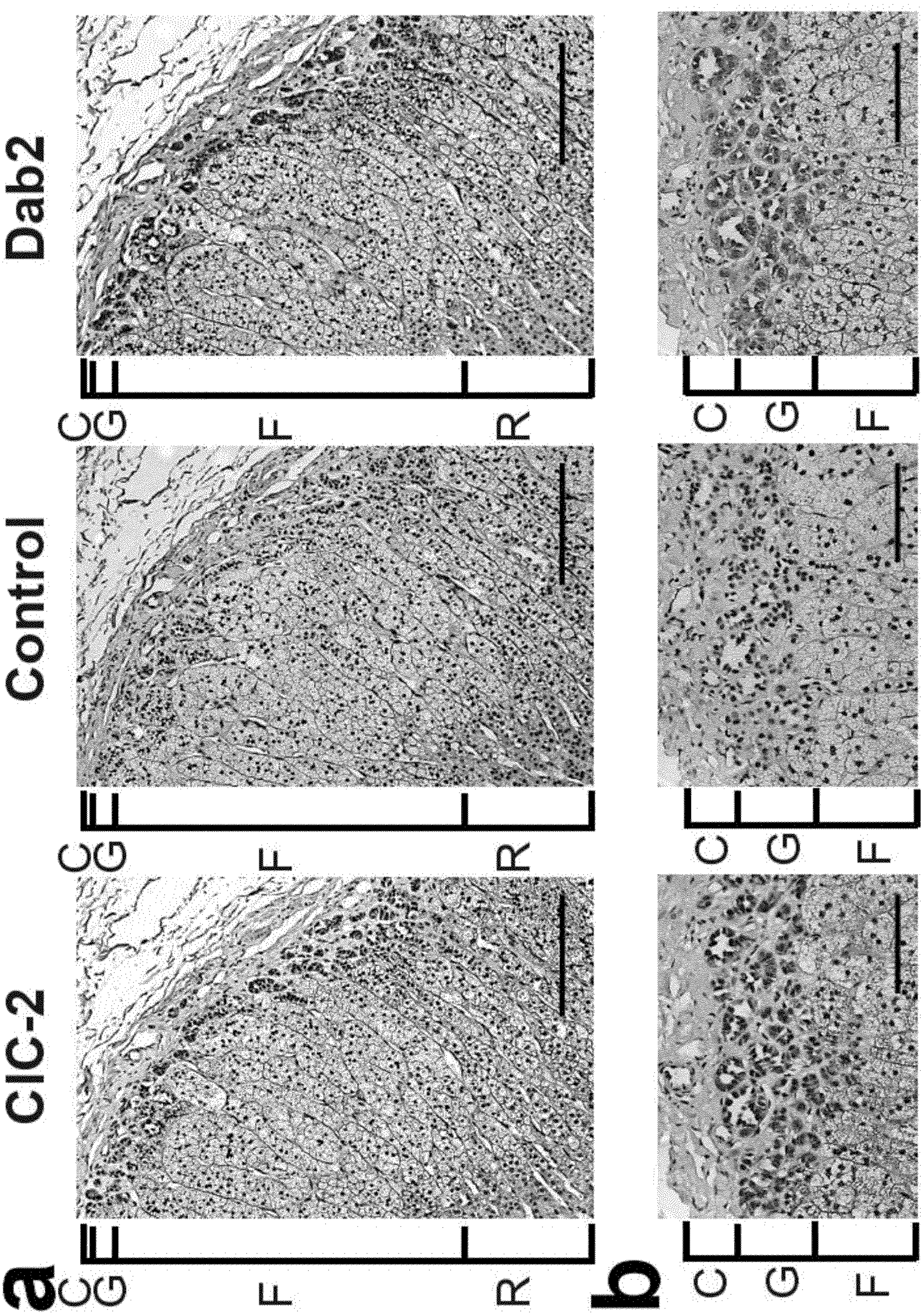

Figure 3:

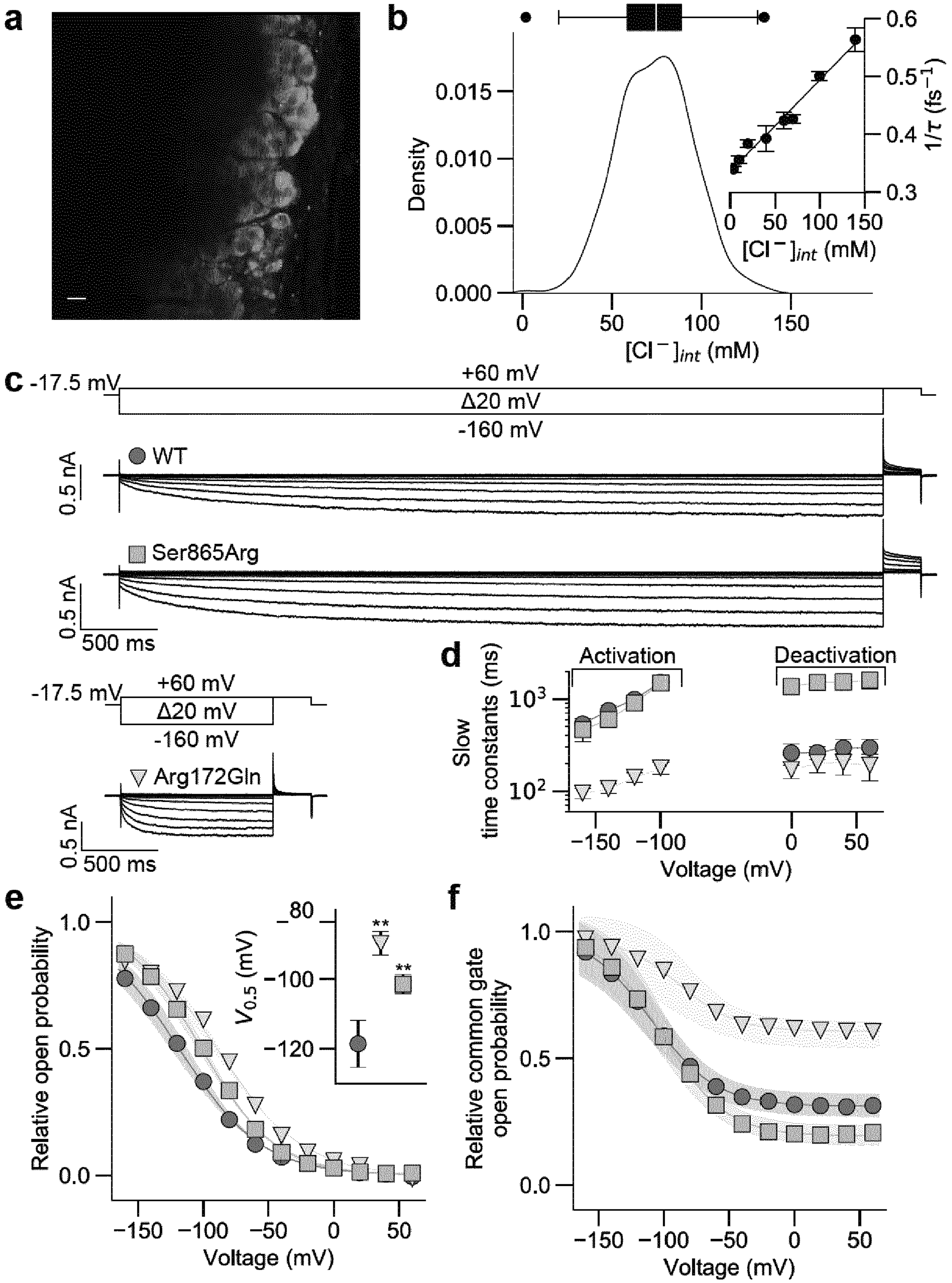
a
b
Density
0.015
0.010
0.005
0.000
0
50
100
150
[Cl⁻]int (mM)
0.6
0.5
0.4
0.3
1/τ (fs⁻¹)
c
-17.5 mV
+60 mV
Δ20 mV
-160 mV
WT
0.5 nA
Ser865Arg
500 ms
Arg172Gln
d
Activation
Deactivation
Slow time constants (ms)
Voltage (mV)
e
Relative open probability
V0.5 (mV)
-80
-100
-120
**
f
Relative common gate open probability
1.0
0.5
0.0
-150
-100
-50

Figure 4:

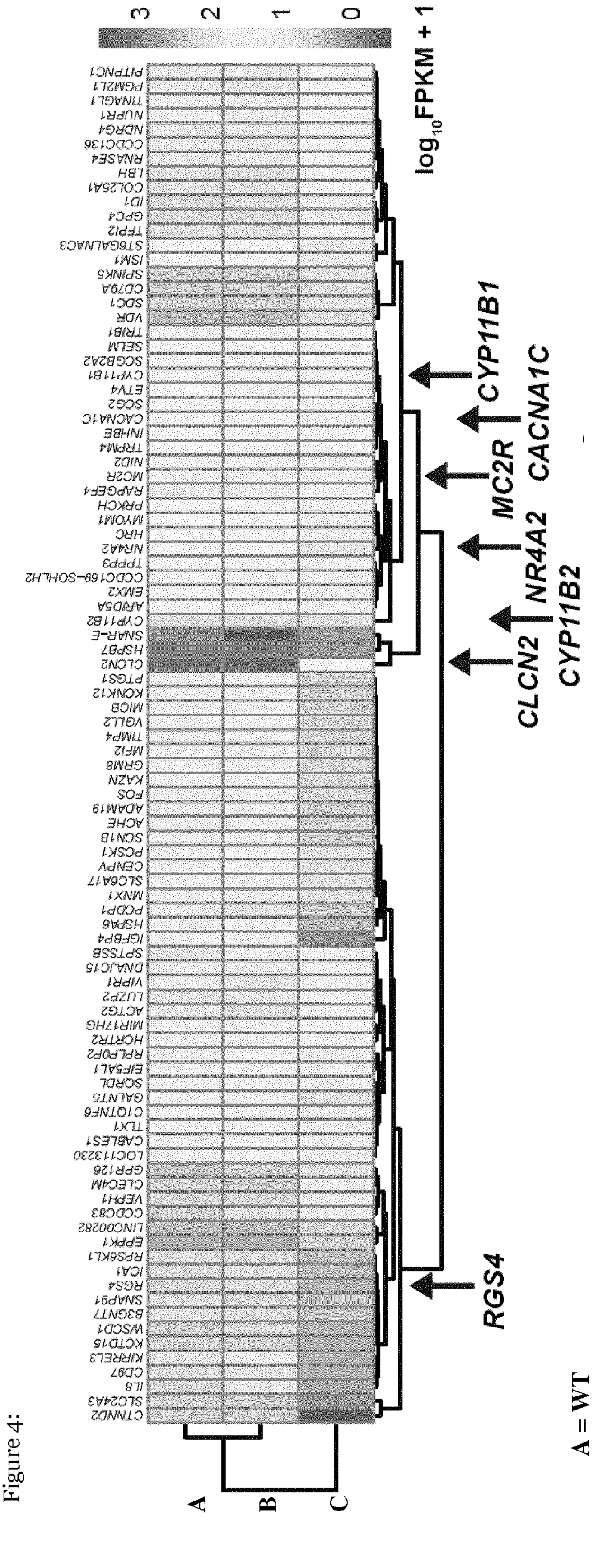


A = WT

B = p.Arg172Gln

C = control

Figure 4 cont.:

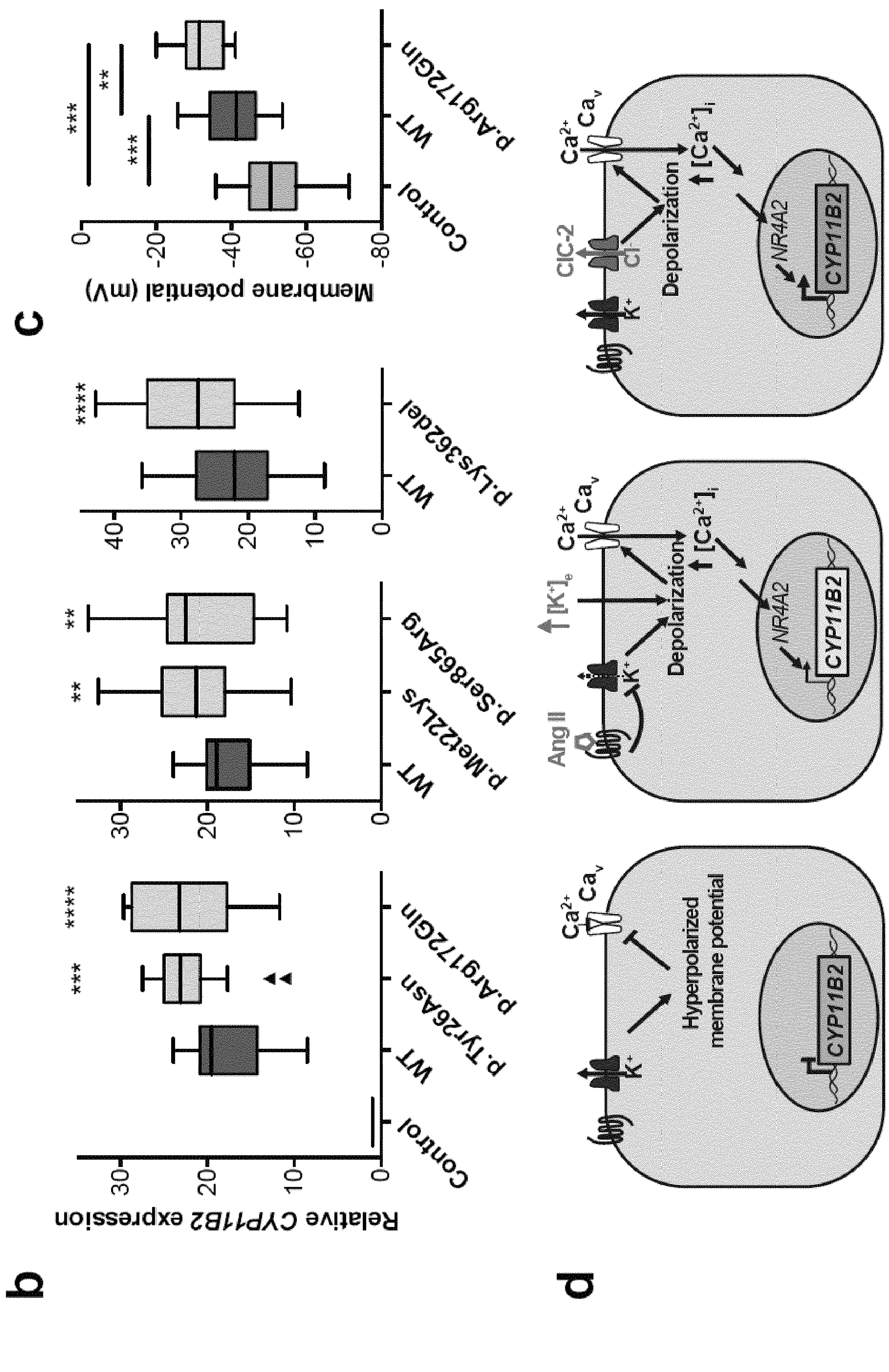

Europäisches Patentamt
European Patent Office
Office européen des brevets

# EUROPEAN SEARCH REPORT

Application Number
EP 17 20 9972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "PREDICTED: Canis familiaris similar to Chloride channel protein 2 (ClC - Nucleotide - NCBI", , 31 August 2005 (2005-08-31), XP055450555, Retrieved from the Internet: URL:https://www.ncbi.nlm.nih.gov/nuccore/XM_854376.1?report=genbank [retrieved on 2018-02-13] * the whole document * | 1-4, 10-14 | INV. C12Q1/6883 C07K14/435 C07K14/705 |
| X | Anonymous: "CLCN2 - Chloride voltage-gated channel 2 - Sarcophilus harrisii (Tasmanian devil) - CLCN2 gene & protein", , 16 November 2011 (2011-11-16), XP055445848, Retrieved from the Internet: URL:http://www.uniprot.org/uniprot/G3VUN5 [retrieved on 2018-01-30] * the whole document * | 1,2,4, 10-14 | TECHNICAL FIELDS SEARCHED (IPC): C12Q C07K |
| X | Anonymous: "PREDICTED: Neolamprologus brichardi chloride channel protein 2-like (L - Nucleotide - NCBI", , 10 February 2014 (2014-02-10), XP055450911, Retrieved from the Internet: URL:https://www.ncbi.nlm.nih.gov/nuccore/584012763 [retrieved on 2018-02-14] * the whole document * | 1,2,4, 10-14 | |

-/--

The present search report has been drawn up for all claims

| Place of search | Date of completion of the search | Examiner |
|---|---|---|
| Munich | 28 February 2018 | Ulbrecht, Matthias |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 17 20 9972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "PREDICTED: Ovis aries chloride channel, voltage-sensitive 2 (CLCN2), t - Nucleotide - NCBI",<br>,<br>17 December 2015 (2015-12-17), XP055450920,<br>Retrieved from the Internet:<br>URL:https://www.ncbi.nlm.nih.gov/nuccore/965917697<br>[retrieved on 2018-02-14]<br>* the whole document *<br>----- | 1,2,4, 10-14 | |
| X | WO 2004/039979 A1 (UNIV BONN [DE]; HEILS ARMIN [DE]; HAUG KARSTEN [DE])<br>13 May 2004 (2004-05-13)<br>* abstract *<br>* page 11, line 19 *<br>* page 12, line 1 - page 14, line 10 *<br>* page 16, line 1 - line 32 *<br>* page 30, line 19 - page 31, line 9 *<br>* page 32, line 4 - page 34, line 22 *<br>* page 35, line 1 - page 36, line 31 *<br>* page 37, line 13 - line 30 *<br>* page 39, line 26 - page 48, line 9 *<br>* page 52, line 29 - page 53, line 16 *<br>* SEQ ID No1 to SEQ ID No21; sequences 1-21 *<br>----- | 1,4,10, 11,13,14 | TECHNICAL FIELDS SEARCHED (IPC) |
| X | STOGMANN E ET AL: "Mutations in the CLCN2 gene are a rare cause of idiopathic generalized epilepsy syndromes",<br>NEUROGENETICS, SPRINGER, BERLIN, DE,<br>vol. 7, no. 4, 24 August 2006 (2006-08-24), pages 265-268, XP019431635,<br>ISSN: 1364-6753, DOI: 10.1007/S10048-006-0057-X<br>* page 266, column 2 *<br>-----<br>-/-- | 1,4,13, 14 | |

The present search report has been drawn up for all claims

| Place of search | Date of completion of the search | Examiner |
|---|---|---|
| Munich | 28 February 2018 | Ulbrecht, Matthias |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 20 9972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/127126 A1 (LIFTON RICHARD P [US] ET AL) 8 May 2014 (2014-05-08)<br>* paragraph [0006] *<br>* paragraph [0007] - paragraph [0010] *<br>* claims 19-30 *<br>----- | 5-9 | |
| | | | TECHNICAL FIELDS SEARCHED (IPC) |

The present search report has been drawn up for all claims

2

| Place of search | Date of completion of the search | Examiner |
|---|---|---|
| Munich | 28 February 2018 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 17 20 9972

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2018

| Patent document cited in search report | Publication date | Patent family member(s) | Publication date |
|---|---|---|---|
| WO 2004039979 A1 | 13-05-2004 | AU 2003276217 A1 | 25-05-2004 |
| | | WO 2004039979 A1 | 13-05-2004 |
| US 2014127126 A1 | 08-05-2014 | US 2014127126 A1 | 08-05-2014 |
| | | WO 2012094394 A2 | 12-07-2012 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MONTICONE, S. et al.** *J Am Coll Cardiol,* 2017, vol. 69, 1811-1820
- **STOWASSER, M. et al.** Familial hyperaldosteronism type II: five families with a new variety of primary aldosteronism. *Clin Exp Pharmacol Physiol,* 1992, vol. 19, 319-22
- **THIEMANN.** *Nature,* 1992, vol. 365, 57-60
- **CID.** *Hum. Mol. Genet.,* 1995, vol. 4, 407-413
- **SMITH.** *J. Neurosci.,* 1995, vol. 15, 4057-4067
- **SIK.** *Neuroscience,* 2000, vol. 101, 51-65
- **SANDER, J.D. et al.** CRISPR-Cas systems for editing, regulating and targeting genomes. *Nature Biotechnology,* 2014, vol. 32, 347-355
- **HSU, P.D. et al.** Development and applications of CRISPR-Cas9 for genome engineering. *Cell,* 2014, vol. 157 (6), 1262-1278
- **STOWASSER, M. et al.** *Clin Exp Pharmacol Physiol,* 1992, vol. 19, 319-22
- **MONTICONE, S. et al.** Prevalence and Clinical Manifestations of Primary Aldosteronism Encountered in Primary Care Practice. *J Am Coll Cardiol,* 2017, vol. 69, 1811-1820
- Collaboration, N.C.D.R.F. Worldwide trends in blood pressure from 1975 to 2015: a pooled analysis of 1479 population-based measurement studies with 19.1 million participants. *Lancet,* 2016
- **LIM, S.S. et al.** A comparative risk assessment of burden of disease and injury attributable to 67 risk factors and risk factor clusters in 21 regions, 1990-2010: a systematic analysis for the Global Burden of Disease Study 2010. *Lancet,* 2012, vol. 380, 2224-60
- **FUNDER, J.W. et al.** The Management of Primary Aldosteronism: Case Detection, Diagnosis, and Treatment: An Endocrine Society Clinical Practice Guideline. *J Clin Endocrinol Metab,* 2016, vol. 101, 1889-916
- **CHOI, M. et al.** K+ channel mutations in adrenal aldosterone-producing adenomas and hereditary hypertension. *Science,* 2011, vol. 331, 768-72
- **SCHOLL, U.I. et al.** Somatic and germline CACNA1D calcium channel mutations in aldosterone-producing adenomas and primary aldosteronism. *Nat Genet,* 2013, vol. 45, 1050-4
- **AZIZAN, E.A. et al.** Somatic mutations in ATP1A1 and CACNA1D underlie a common subtype of adrenal hypertension. *Nat Genet,* 2013, vol. 45, 1055-60
- **BEUSCHLEIN, F. et al.** Somatic mutations in ATP1A1 and ATP2B3 lead to aldosterone-producing adenomas and secondary hypertension. *Nat Genet,* 2013, vol. 45, 440-4
- **FERNANDES-ROSA, F.L. et al.** Genetic spectrum and clinical correlates of somatic mutations in aldosterone-producing adenoma. *Hypertension,* 2014, vol. 64, 354-61
- **LIFTON, R.P. et al.** A chimaeric 11 beta-hydroxylase/aldosterone synthase gene causes glucocorticoid-remediable aldosteronism and human hypertension. *Nature,* 1992, vol. 355, 262-5
- **SCHOLL, U.I. et al.** Hypertension with or without adrenal hyperplasia due to different inherited mutations in the potassium channel KCNJ5. *Proc Natl Acad Sci U S A,* 2012, vol. 109, 2533-8
- **SCHOLL, U.I. et al.** Recurrent gain of function mutation in calcium channel CACNA1H causes early-onset hypertension with primary aldosteronism. *Elife,* vol. 4, 2015
- **KORAH, H.E. ; SCHOLL, U.I.** An Update on Familial Hyperaldosteronism. *Horm Metab Res,* 2015, vol. 47, 941-6
- **SPAT, A. ; HUNYADY, L.** Control of aldosterone secretion: a model for convergence in cellular signaling pathways. *Physiol Rev,* 2004, vol. 84, 489-539
- **CARSS, K.J. ; STOWASSER, M. ; GORDON, R.D. ; O'SHAUGHNESSY.** K.M. Further study of chromosome 7p22 to identify the molecular basis of familial hyperaldosteronism type II. *J Hum Hypertens,* 2011, vol. 25, 560-4
- **DONG, C. et al.** Comparison and integration of deleteriousness prediction methods for nonsynonymous SNVs in whole exome sequencing studies. *Hum Mol Genet,* 2015, vol. 24, 2125-37
- **GENIN, E. ; TULLIO-PELET, A. ; BEGEOT, F. ; LYONNET, S. ; ABEL, L.** Estimating the age of rare disease mutations: the example of Triple-A syndrome. *J Med Genet,* 2004, vol. 41, 445-9
- **DOGAN, R.I. ; GETOOR, L. ; WILBUR, W.J. ; MOUNT, S.M.** SplicePort--an interactive splice-site analysis tool. *Nucleic Acids Res,* 2007, vol. 35, W285-91
- **THIEMANN, A. ; GRUNDER, S. ; PUSCH, M. ; JENTSCH, T.J.** A chloride channel widely expressed in epithelial and non-epithelial cells. *Nature,* 1992, vol. 356, 7-60

- **UNTIET, V. et al.** Glutamate transporter-associated anion channels adjust intracellular chloride concentrations during glial maturation. *Glia,* 2017, vol. 65, 388-400
- **HU, C. ; RUSIN, C.G. ; TAN, Z. ; GUAGLIARDO, N.A. ; BARRETT, P.Q.** Zona glomerulosa cells of the mouse adrenal cortex are intrinsic electrical oscillators. *J Clin Invest,* 2012, vol. 122, 2046-53
- **JEWORUTZKI, E. et al.** GlialCAM, a protein defective in a leukodystrophy, serves as a ClC-2 Cl(-) channel auxiliary subunit. *Neuron,* 2012, vol. 73, 951-61
- **STOLTING, G. et al.** Regulation of ClC-2 gating by intracellular ATP. *Pflugers Arch,* 2013, vol. 465, 1423-37
- **NIEMEYER, M.I. ; CID, L.P. ; ZUNIGA, L. ; CATALAN, M. ; SEPULVEDA, F.V.** A conserved pore-lining glutamate as a voltage- and chloride-dependent gate in the ClC-2 chloride channel. *J Physiol,* 2003, vol. 553, 873-9
- **JENTSCH, T.J.** Discovery of CLC transport proteins: cloning, structure, function and pathophysiology. *J Physiol,* 2015, vol. 593, 4091-109
- **RAINEY, W.E. ; BIRD, I.M. ; MASON, J.I.** The NCI-H295 cell line: a pluripotent model for human adrenocortical studies. *Mol Cell Endocrinol,* 1994, vol. 100, 45-50
- **BASSETT, M.H. ; SUZUKI, T. ; SASANO, H. ; WHITE, P.C. ; RAINEY, W.E.** The orphan nuclear receptors NURR1 and NGFIB regulate adrenal aldosterone production. *Mol Endocrinol,* 2004, vol. 18, 279-90
- **ROMERO, D.G. et al.** Regulators of G-protein signaling 4 in adrenal gland: localization, regulation, and role in aldosterone secretion. *J Endocrinol,* 2007, vol. 194, 429-40
- **GARCIA-OLIVARES, J. et al.** Gating of human ClC-2 chloride channels and regulation by carboxy-terminal domains. *J Physiol,* 2008, vol. 586, 5325-36
- **TAUBER, P. et al.** Cellular Pathophysiology of an Adrenal Adenoma-Associated Mutant of the Plasma Membrane Ca(2+)-ATPase ATP2B3. *Endocrinology,* 2016, vol. 157, 2489-99
- **LAFFERTY, A.R. et al.** A novel genetic locus for low renin hypertension: familial hyperaldosteronism type II maps to chromosome 7 (7p22). *J Med Genet,* 2000, vol. 37, 831-5
- **DEPIENNE, C. et al.** Brain white matter oedema due to ClC-2 chloride channel deficiency: an observational analytical study. *Lancet Neurol,* 2013, vol. 12, 659-68
- **BLANZ, J. et al.** Leukoencephalopathy upon disruption of the chloride channel ClC-2. *J Neurosci,* 2007, vol. 27, 6581-9
- **STOWASSER, M. et al.** Clinical, biochemical and genetic approaches to the detection of familial hyperaldosteronism type I. *J Hypertens,* 1995, vol. 13, 1610-3
- **CHORVATOVA, A. ; GENDRON, L. ; BILODEAU, L. ; GALLO-PAYET, N. ; PAYET, M.D.** A Ras-dependent chloride current activated by adrenocorticotropin in rat adrenal zona glomerulosa cells. *Endocrinology,* 2000, vol. 141, 684-92
- **TORPY, D.J. et al.** Familial hyperaldosteronism type II: description of a large kindred and exclusion of the aldosterone synthase (CYP11B2) gene. *J Clin Endocrinol Metab,* 1998, vol. 83, 3214-8
- **KRUMM, N. et al.** Excess of rare, inherited truncating mutations in autism. *Nat Genet,* 2015, vol. 47, 582-8
- **VERKMAN, A.S.** Development and biological applications of chloride-sensitive fluorescent indicators. *Am J Physiol,* 1990, vol. 259, C375-88
- **KANEKO, H. ; PUTZIER, I. ; FRINGS, S. ; KAUPP, U.B. ; GENSCH, T.** Chloride accumulation in mammalian olfactory sensory neurons. *J Neurosci,* 2004, vol. 24, 7931-8
- **BEVENSEE, M.O. ; APKON, M. ; BORON, W.F.** Intracellular pH regulation in cultured astrocytes from rat hippocampus. II. Electrogenic Na/HCO3 cotransport. *J Gen Physiol,* 1997, vol. 110, 467-83
- **CHAO, A.C. ; DIX, J.A. ; SELLERS, M.C. ; VERKMAN, A.S.** Fluorescence measurement of chloride transport in monolayer cultured cells. Mechanisms of chloride transport in fibroblasts. *Biophys J,* 1989, vol. 56, 1071-81
- **ACCARDI, A. ; PUSCH, M.** Fast and slow gating relaxations in the muscle chloride channel CLC-1. *J Gen Physiol,* 2000, vol. 116, 433-44
- **DE SANTIAGO, J.A. ; NEHRKE, K. ; ARREOLA, J.** Quantitative analysis of the voltage-dependent gating of mouse parotid ClC-2 chloride channel. *J Gen Physiol,* 2005, vol. 126, 591-603
- **RHEE, J.S. ; EBIHARA, S. ; AKAIKE, N.** Gramicidin perforated patch-clamp technique reveals glycine-gated outward chloride current in dissociated nucleus solitarii neurons of the rat. *J Neurophysiol,* 1994, vol. 72, 1103-8
- **KIM, D. et al.** TopHat2: accurate alignment of transcriptomes in the presence of insertions, deletions and gene fusions. *Genome Biol,* 2013, vol. 14, R36
- **TRAPNELL, C. et al.** Differential analysis of gene regulation at transcript resolution with RNA-seq. *Nat Biotechnol,* 2013, vol. 31, 46-53
- **SAMOCHA, K.E. et al.** A framework for the interpretation of de novo mutation in human disease. *Nat Genet,* 2014, vol. 46, 944-50
- **ZOU, J. et al.** Quantifying unobserved protein-coding variants in human populations provides a roadmap for large-scale sequencing projects. *Nat Commun,* 2016, vol. 7, 13293

• **LEK, M. et al.** Analysis of protein-coding genetic variation in 60,706 humans. *Nature,* 2016, vol. 536, 285-91